# EUROPEAN PATENT SPECIFICATION

(11) **EP 3 651 848 B1**
(45) Date of publication and mention of the grant of the patent: **15.05.2024**
(21) Application number: 18743239.8
(22) Date of filing: 27.06.2018
(51) Int. Cl.: A61N 1/36, A61B 5/00, A61N 1/08, A61N 1/05, A61N 1/375, A61B 5/055

(54) **METHODS, IMPLANTABLE MEDICAL LEADS, AND RELATED SYSTEMS TO MONITOR AND LIMIT TEMPERATURE CHANGES IN PROXIMITY TO ELECTRODES**
VERFAHREN, IMPLANTIERBARE MEDIZINISCHE LEITUNGEN UND ZUGEHÖRIGE SYSTEME ZUR ÜBERWACHUNG UND BEGRENZUNG VON TEMPERATURVERÄNDERUNGEN IN DER NÄHE VON ELEKTRODEN
MÉTHODES, SONDES MÉDICALES IMPLANTABLES ET SYSTÈMES ASSOCIÉS POUR SURVEILLER ET LIMITER DES VARIATIONS DE TEMPÉRATURE À PROXIMITÉ D'ÉLECTRODES

(30) Priority: 08.07.2017 US 201762530153 P
(43) Date of publication of application: 20.05.2020
(73) Proprietor: Medtronic, Inc., Minneapolis, Minnesota 55432 (US)
(72) Inventor: STEM, Bryan D., Coon Rapids, Minnesota 55433 (US)
(74) Representative: Zimmermann & Partner Patentanwälte mbB
(86) International application number: PCT/US2018/039786
(87) International publication number: WO 2019/013977

(56) References cited:
- DE-A1- 19 738 543
- US-A1- 2006 025 820
- US-A1- 2011 066 028
- US-A1- 2011 270 362
- US-A1- 2016 199 639

## Description

### TECHNICAL FIELD

Embodiments relate to implantable medical leads and systems. More particularly, embodiments relate to implantable medical leads and systems where a temperature change in proximity to an electrode is being monitored so that the temperature change at the electrode may be limited when necessary.

### BACKGROUND

Implantable medical leads include electrodes at a distal end in order to provide electrical stimulation to tissue at a target site within the body and/or to provide sensing of physiological signals at the target site. The implantable medical leads have a proximal end that is coupled to an implantable medical device (IMD) that performs the electrical stimulation and/or physiological sensing. Electrical conductors extend through the implantable medical leads from the IMD, which is positioned at a convenient implantation site, to the electrodes located at the target site.

During procedures such as a magnetic resonance imaging (MRI) scan where a level of radio frequency electromagnetic energy much greater than in normal ambient conditions is present, an electrical current is induced onto the electrical conductors of the lead. This electrical current passes through the electrode to generate heating of the electrode to tissue interface, and this heating can be potentially dangerous to the patient having the implantable medical system that includes the IMD and one or more leads. Various techniques may be used to reduce the degree of heating. One example is to include a shield within the lead that surrounds the electrical conductors. Other examples include increasing the impedance of the electrical conductors via chokes and the like.

These techniques have been proven effective for implantable medical systems where the lead is routed well below the surface of the body of the patient. However, in some cases, the lead may be routed near the surface, such as for peripheral nerve stimulation therapy. In such a case, the shallow depth of the lead within the body results in exposure to higher levels of the RF energy. These higher levels may exceed the capabilities of a lead to prevent excessive heating at the electrode to tissue interface for a lead designed originally to lessen the effects of RF energy for a deeper type of implantation.

Document US 2016199639 A1 discloses an implantable medical leads include electrodes at a distal end in order to provide electrical stimulation to tissue at a target site.

### SUMMARY

Embodiments address issues such as these and others by providing for monitoring of temperature changes within an implantable medical lead nearby an electrode in order to limit the degree of heating. A temperature sensor may be positioned within the lead and in proximity to the electrode and may have a signal path back to a temperature probe processor. When the monitored temperature as determined by the temperature probe processor exceeds a threshold, an action may be taken to limit the degree of heating. For instance, the implantable medical device or separate probe processing device may trigger an alarm via telemetry that alerts an MRI technician to stop the MRI scan or may submit a machine instruction via telemetry that stops the MRI scan. The implantable medical device may also trigger a series switch in the conduction path to open to attempt to block conduction of the RF energy and/or may trigger a shunt in parallel to the conduction path to become active to divert some of the RF energy away from the electrode being heated.

Embodiments provide a method of reducing heating at an electrode on a lead of an implantable medical system during an MRI scan. The method involves monitoring a temperature at the electrode during an MRI scan and upon detecting that the temperature being monitored exceeds a threshold, then shunting electrical current within a conductor of the lead to a heat sink externally located on the implantable medical system.

Embodiments provide method of reducing heating at an electrode on a lead of an implantable medical system during an MRI scan. The method involves monitoring a temperature at the electrode during an MRI scan and upon detecting that the temperature being monitored exceeds a threshold, then blocking electrical current within a stimulation conductor of the lead by creating an open circuit with a control conductor coupled to a switch that is connected in series with the stimulation conductor.

Embodiments provide an implantable medical system that includes a heat sink and an implantable medical device that includes a stimulation engine and an electrical connector that is electrically coupled to an output of the stimulation engine. The system further includes an implantable medical lead that includes a lead body, a proximal contact on a proximal end of the lead body, the proximal contact being electrically coupled to the electrical connector, and a distal electrode on a distal end of the lead body. The lead further includes an electrical conductor that electrically interconnects the proximal contact to the distal electrode, with the proximal contact electrically coupled to the electrical connector so as to create a conduction path from the electrical connector to the distal electrode and a temperature sensor within the lead body and adjacent the distal electrode. The lead also includes a signal path extending proximally from the temperature sensor through the lead body. The system further includes a switch having a control input, a first connection electrically connected to the heat sink, and a second connection electrically connected to the conduction path so as to be in parallel with a portion of the conduction path that extends from the second connection to the distal electrode such that when the switch is closed the conduction path is shunted to the heat sink through the switch. The system also includes a controller in communication with the control input of the switch and in communication with the signal path of the temperature sensor such that the controller receives a signal representative of temperature from the temperature sensor and closes the switch when the temperature as represented by the received signal exceeds a threshold.

Embodiments provide an implantable medical system that includes an implantable medical device that includes a stimulation engine and an electrical connector that is electrically coupled to an output of the stimulation engine. The system further includes an implantable medical lead that that includes a lead body, a proximal contact on a proximal end of the lead body, the proximal contact being electrically coupled to the electrical connector, a distal electrode on a distal end of the lead body, and an electrical conductor that electrically interconnects the proximal contact to the distal electrode, with the proximal contact electrically coupled to the electrical connector so as to create a conduction path from the electrical connector to the distal electrode. The lead further includes a temperature sensor within the lead body and adjacent the distal electrode and a signal path extending proximally from the temperature sensor through the lead body. The system further includes a switch having a control input and being connected in series in the conduction path such that when the switch is opened the conduction path is an open circuit at the switch. The system also includes a controller in communication with the control input of the switch and in communication with the signal path of the temperature sensor such that the controller receives a signal representative of temperature from the temperature sensor and opens the switch when the temperature as represented by the received signal exceeds a threshold.

### DESCRIPTION OF THE DRAWINGS

FIG. 1 shows an operating environment for various embodiments of an implantable medical system.
FIG. 2 shows an embodiment of an implantable medical device and an implantable medical lead coupled together and providing a fiber optic cable as a signal path for a temperature sensor.
FIG. 3 shows an embodiment of an implantable medical device and an implantable medical lead coupled together and providing an electrical conductor and header connection as a signal path for a temperature sensor.
FIG. 4 shows an embodiment of an implantable medical device and an implantable medical lead coupled together and providing a signal path for a temperature sensor that terminates to a separate probe processing unit attached to the implantable medical device.
FIG. 5 shows an embodiment of an implantable medical device and an implantable medical lead coupled together and providing a signal path for a temperature sensor that terminates to a separate probe processing unit attached to the implantable medical lead.
FIG. 6 shows a lateral cross-sectional view of the separate probe processing unit and lead of the embodiment of FIG. 5.
FIG. 7 shows an embodiment of a distal end of the lead where a temperature sensor is positioned within a distal electrode.
FIG. 8 shows an embodiment of a distal end of the lead where a temperature sensor is positioned longitudinally adjacent to a distal electrode.
FIG. 9 shows an embodiment where the temperature sensor is affixed to an interior surface of an electrode.
FIG. 10 shows an embodiment where the temperature sensor is encapsulated in a conductive filler material that may be positioned within an electrode or elsewhere within the lead.
FIG. 11 shows an embodiment of a distal end of the lead where a temperature sensor is positioned longitudinally adjacent to a distal electrode and within a separate conductive ring.
FIG. 12 shows a lateral cross-sectional view of the temperature sensor within the separate ring.
FIG. 13 shows a perspective view of the embodiment of FIG. 12 with the temperature sensor within the separate ring.
FIG. 14 shows an embodiment of a distal end of the lead where a temperature sensor is positioned longitudinally adjacent to a distal electrode and within a conductive filler material at the tip of the lead.
FIG. 15 shows an embodiment of a distal end of the lead where a temperature sensor is positioned longitudinally adjacent to a distal electrode and within a metal tip of the lead.
FIG. 16 shows a perspective view of the embodiment of FIG. 15 with the temperature sensor positioned within the metal tip.
FIG. 17 shows an embodiment of an implantable medical system where the signal path for the temperature sensor is an electrical conductor that is also coupled to the electrode.
FIG. 18 shows a set of operations that an implantable medical device or separate probe processing device may perform to monitor the temperature change at the electrode and limit the temperature increase when using a fiber optic cable or electrical conductor not shared with an electrode.
FIG. 19 shows a set of operations that an implantable medical device may perform to monitor the temperature change at the electrode and limit the temperature increase when the temperature probe shares an electrical conductor with the electrode.
FIG. 20 shows an embodiment of an implantable medical device and an implantable medical lead coupled together and providing a fiber optic cable as a signal path for a temperature sensor where the lead includes a shunt control conductor, shunt conductor, and a shunt switch block while a shunt heat sink is present at the device.
FIG. 21 shows an embodiment of an implantable medical device and an implantable medical lead coupled together and providing an electrical conductor and header connection as a signal path for a temperature sensor where the lead includes a shunt control conductor, shunt conductor, and a shunt switch block while a shunt heat sink is present at the device.
FIG. 22 shows an embodiment of an implantable medical device and an implantable medical lead coupled together and providing a signal path for a temperature sensor that terminates to a separate probe processing unit attached to the implantable medical device where the lead includes a shunt control conductor, shunt conductor, and a shunt switch block while a shunt heat sink is present at the separate probe processing unit.
FIG. 23 shows an embodiment of an implantable medical device and an implantable medical lead coupled together and providing a signal path for a temperature sensor that terminates to a separate probe processing unit attached to the implantable medical lead where the lead includes a shunt control conductor, shunt conductor, and a shunt switch block while a shunt heat sink is present at the separate probe processing unit.
FIG. 24 shows a portion of an embodiment of an implantable medical lead that provides a signal path for a temperature sensor and where the lead includes a shunt control conductor, shunt conductor, and a shunt switch block while a shunt heat sink is present on the lead.
FIG. 25 shows an embodiment of an implantable medical device and an implantable medical lead coupled together and providing a fiber optic cable as a signal path for a temperature sensor where the lead contains a control conductor to a series switch block.
FIG. 26 shows an embodiment of an implantable medical device and an implantable medical lead coupled together and providing an electrical conductor and header connection as a signal path for a temperature sensor where the lead contains a control conductor to a series switch block.
FIG. 27 shows an embodiment of an implantable medical device and an implantable medical lead coupled together and providing a signal path for a temperature sensor that terminates to a separate probe processing unit attached to the implantable medical device where the lead contains a control conductor to a series switch block.
FIG. 28 shows an embodiment of an implantable medical device and an implantable medical lead coupled together and providing a signal path for a temperature sensor that terminates to a separate probe processing unit attached to the implantable medical lead where the lead contains a control conductor to a series switch block.
FIG. 29 shows an embodiment of a probe processing unit, like that of FIGS. 22 and 23, where the probe processing unit includes a shunt switch block.

### DETAILED DESCRIPTION

Embodiments provide for temperature monitoring at an electrode of an implantable medical lead to allow detection of excessive heating and to further allow for action to be taken to limit further heating. The temperature sensor is positioned within the lead either within or adjacent to an electrode. A signal path for the temperature sensor may be of various forms such as an electrical conductor or a fiber optic cable. The signal path may lead to a connection within a header of an IMD or may lead to a separate probe processing unit, and the probe processing unit may be attached to either the IMD or to the lead.

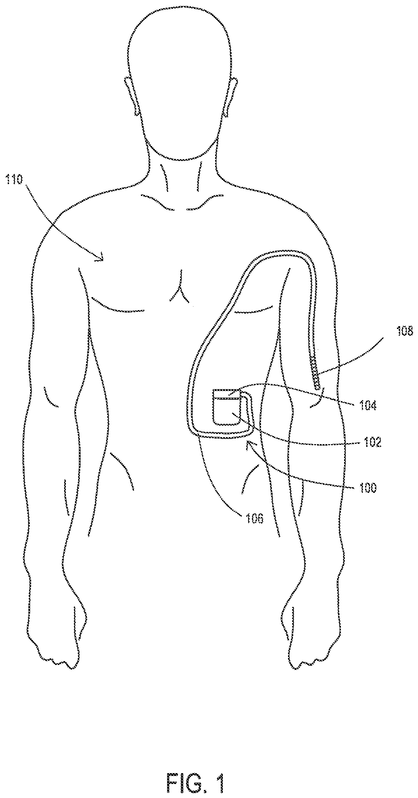

FIG. 1 shows an example of an implantable medical system 100 that includes an IMD 102 with a header 104 and a lead 106 coupled to the header 104 of the IMD 102. Electrodes 108 are positioned at a distal end of the lead 106 and are located at a target site within the body of a patient 110. In this example, the target site is within the arm of the patient 110 to provide peripheral nerve stimulation, but it will be appreciated that the target site may be located in other areas of the body of the patient 110.

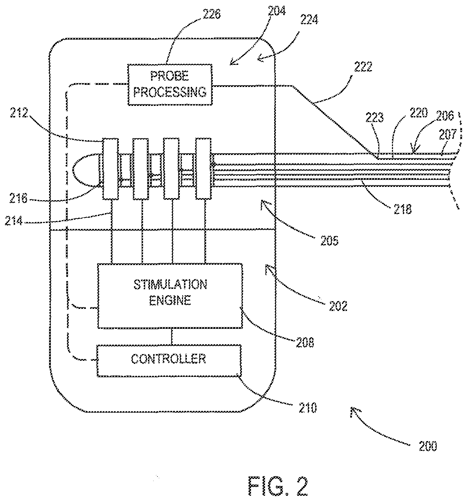

FIG. 2 shows an embodiment of an implantable medical system 200 where an IMD 202 includes a header portion 204 that includes a first bore 205 where a proximal end of a lead 206 is inserted. The header portion 204 also includes a second bore 224 where a proximal portion 222 of a fiber optic cable is inserted. The proximal portion 222 exits from a body 207 of the lead 206 at an exit point 223. The remaining portion 220 extends distally through the body 207 of the lead 206 to the temperature sensor (not shown in this view).

The proximal portion 222 engages a port of a probe processing unit 226 that is positioned within the header portion 204. The probe processing unit 226 may be a conventional temperature sensing unit with a fiber optic port suitable for receiving the fiber optic cable portion 222. The probe processing unit 226 may have an output that is electrically coupled to a stimulation engine 208 and/or to a controller 210 of the IMD 202. The probe processing unit 226 may then output a signal representative of the measured temperature to a controller 210, and the controller 210 may then compare the temperature to a threshold and take further action. For instance, the controller 210 may cause the stimulation engine 208 or other circuit to operate a switch to shunt energy to the device casing or other heat sink or to operate a switch near the electrodes to open the circuit or shunt the energy elsewhere. The controller may additionally or alternatively telemeter an alarm or machine instruction to an external device to cause the MRI scan to be altered. The controller 210 has telemetry circuitry either integral or coupled thereto which allows communication with external devices. Alternatively, the probe processing unit may perform additional logic such as comparing the temperature to a threshold and then sending an interrupt signal to the stimulation engine 208 or other circuit to cause further action to be taken such as shunting away the energy to the casing or a heat sink.

The lead 206 also includes one or more electrical conductors 218 that lead to one or more corresponding electrodes on the distal end (not shown in this view) and to one or more corresponding electrical contacts 216 on the proximal end. The header portion 204 includes conventional electrical connectors 212 that are electrically connected via feed through conductors 214 passing from the header portion 204 through a conventional feed through hermetic seal to the stimulation engine 208.

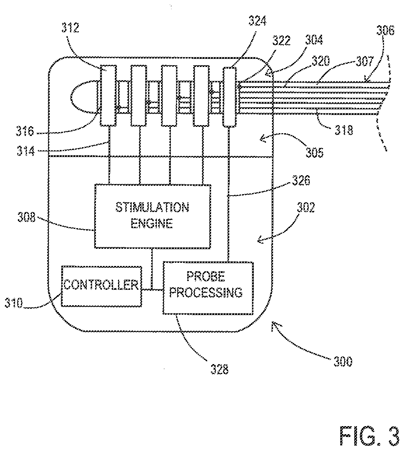

FIG. 3 shows an embodiment of an implantable medical system 300 where an IMD 302 includes a header portion 304 that includes a first bore 305 where a proximal end of a lead 306 is inserted. The proximal end includes an electrical contact 322 that is dedicated to temperature sensor signals and couples to a dedicated electrical connector 324 of the header portion 304. An electrical conductor 320 that is dedicated to the temperature sensor located at the distal end of the lead (not shown in this view) passes through the lead body 307 and is electrically coupled to the dedicated electrical contact 322. Likewise, an electrical feedthrough conductor 326 passes the electrical signal of the temperature sensor from the electrical connector 324 through a conventional feedthrough hermetic seal to a probe processing unit 328 within the IMD 302.

As with FIG. 2, the probe processing unit 328 may be a conventional temperature sensing unit but in this case has an electrical connection suitable for receiving the electrical signals produced by the temperature sensor connected to the electrical conductor 320. The probe processing unit 328 may have an output that is electrically coupled to a stimulation engine 308 and/or to a controller 310 of the IMD 302. The probe processing unit 328 may then output a signal representative of the measured temperature to the controller 310, and the controller 310 may then compare the temperature to a threshold and take further action such as causing the stimulation engine 308 or other circuit to operate a switch to shunt energy to the device casing or other heat sink or to operate a switch near the electrodes to open the circuit or shunt the energy elsewhere or to telemeter an alarm or machine instruction to an external device to cause the MRI scan to be altered. Alternatively, the probe processing unit 328 may perform additional logic such as comparing the temperature to a threshold and then sending an interrupt signal to the stimulation engine 308 or other circuit to cause further action to be taken such as shunting the energy away.

The lead 306 also includes one or more electrical conductors 318 that lead to one or more corresponding electrodes on the distal end (not shown in this view) and to one or more corresponding electrical contacts 316 on the proximal end. The header portion 304 includes conventional electrical connectors 312 that are electrically connected via feed through conductors 314 passing from the header portion 304 through a conventional feed through hermetic seal to the stimulation engine 308.

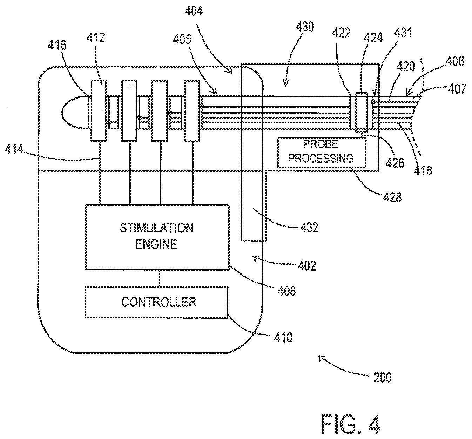

FIG. 4 shows an embodiment of an implantable medical system 400 where a separate probe processing enclosure 430 is attached to the IMD 402. The probe processing enclosure 430 may include various mounting structures 432 such as a flange that compresses against the IMD 402 to fix the enclosure 430 in place. The enclosure 430 includes a bore 431 where a proximal end of an implantable medical lead 406 passes entirely through the enclosure 430 in order to enter a bore 405 of a header portion 404 of the IMD 402.

In this example, the lead 406 includes an electrical contact 422 that is electrically coupled to a dedicated electrical conductor 420 within a lead body 407 of the lead 406. The electrical conductor 420 extends distally to a temperature sensor at a distal end of the lead 406 (not shown in this view). The electrical contact 422 electrically couples to an electrical connector 424 within the bore 431 of the enclosure 430.

A probe processing unit 428 is within the enclosure 430 and therefore separate from the IMD 402. In this example, an electrical conductor 426 electrically couples the probe processing unit 428 to the electrical connector 424. The conductor 426 may pass the electrical signal of the temperature sensor from the electrical connector 424 through a conventional feedthrough hermetic seal of the enclosure 430 to the probe processing unit 428.

While this example shows an electrical conductor 420 and an electrical signal path to the probe processing unit 428, it will be appreciated that a fiber cable could be used with the enclosure 430 and separate probe processing unit. For example, a fiber optic cable within the lead body 407 may exit the lead body distally of the enclosure 430 and then be received within a bore of the enclosure 430, in a similar fashion to the manner in which the header portion 204 receives the fiber cable portion 222 in a bore 205 in FIG. 2.

As with the prior examples, the probe processing unit 428 may be a conventional temperature sensing unit. The probe processing unit 428 may either be configured to receive an electrical signal via the conductor 426 or receive a fiber cable in the alternative. The probe processing unit 428 may have a telemetry circuit to allow the probe processing unit 428 to provide the temperature information to the controller 210. Alternatively, the probe processing unit 428 may perform additional logic such as comparing the temperature to a threshold and then triggering a switch to trigger to shunt the energy away.

The lead 406 also includes one or more electrical conductors 418 that lead to one or more corresponding electrodes on the distal end (not shown in this view) and to one or more corresponding electrical contacts 416 on the proximal end. The header portion 404 includes conventional electrical connectors 412 that are electrically connected via feed through conductors 414 passing from the header portion 404 through a conventional feed through hermetic seal to the stimulation engine 408 which is being controlled by a controller 410.

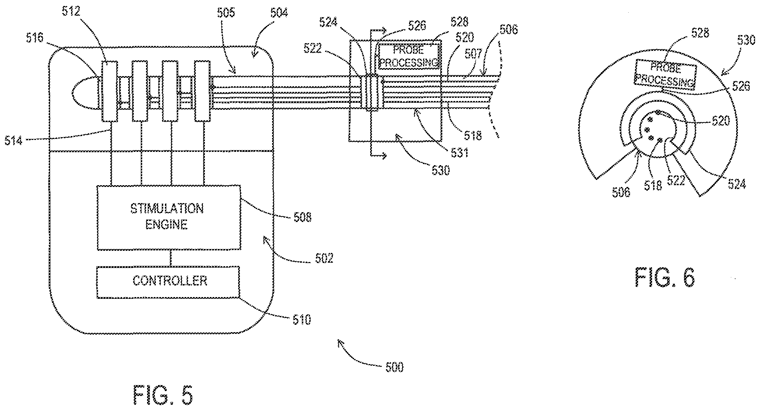

FIG. 5 shows an embodiment of an implantable medical system 500 where a separate probe processing enclosure 530 is attached to a lead 506. The probe processing enclosure 530 may include mounting structures such as an interference fit as shown in the lateral cross-sectional view of FIG. 5 that compresses against the lead 506 to fix the enclosure 530 in place. The enclosure 530 includes a bore 531 where a proximal end of the implantable medical lead 506 passes entirely through the enclosure 530 in order to enter a bore 505 of a header portion 504 of the IMD 502.

In this example, the lead 506 includes an electrical contact 522 that is electrically coupled to a dedicated electrical conductor 520 within a lead body 507 of the lead 506. The electrical conductor 520 extends distally to a temperature sensor at a distal end of the lead 506 (not shown in this view). The electrical contact 522 electrically couples to an electrical connector 524 within the bore 531 of the enclosure 530.

A probe processing unit 528 is within the enclosure 530 and therefore separate from the IMD 502. In this example, an electrical conductor 526 electrically couples the probe processing unit 528 to the electrical connector 524. The conductor 526 may pass the electrical signal of the temperature sensor from the electrical connector 524 through a conventional feedthrough hermetic seal of the enclosure 530 to the probe processing unit 528.

While this example shows an electrical conductor 520 and an electrical signal path to the probe processing unit 528, it will be appreciated that a fiber cable could be used with the enclosure 530 and separate probe processing unit. For example, a fiber optic cable within the lead body 507 may exit the lead body distally of the enclosure 530 and then be received within a bore of the enclosure 530, in a similar fashion to the manner in which the header portion 204 receives the fiber cable portion 222 in a bore 205 in FIG. 2.

As with the prior examples, the probe processing unit 528 may be a conventional temperature sensing unit. The probe processing unit 528 may either be configured to receive an electrical signal via the conductor 526 or receive a fiber cable in the alternative. The probe processing unit 528 may have telemetry to communicate with the controller as described above for the probe processing unit of FIG. 4. Alternatively, the probe processing unit 528 may perform additional logic such as comparing the temperature to a threshold and then trigger a switch to shunt the energy away.

The lead 506 also includes one or more electrical conductors 518 that lead to one or more corresponding electrodes on the distal end (not shown in this view) and to one or more corresponding electrical contacts 516 on the proximal end. The header portion 504 includes conventional electrical connectors 512 that are electrically connected via feed through conductors 514 passing from the header portion 504 through a conventional feed through hermetic seal to the stimulation engine 508 which is being controlled by a controller 510.

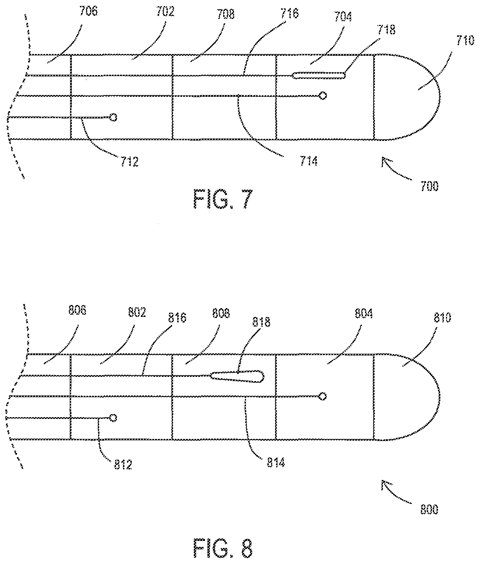

For each of the examples discussed above where an electrical signal is being generated by the temperature sensor, if two conduction paths back to the probe processing unit are required, then the conductor in the lead body may be two separately insulated conductors and that terminate in two separate electrical contacts on the lead. The probe processing unit may then be coupled to two separate electrical connectors that electrically couple to the two separate electrical contacts. As an alternative, one of the conduction paths may be through the body tissue of the patient where the probe processing unit is coupled to a conductive encasement that contacts the body tissue to complete the conduction path to the probe processing unit. FIG. 7 shows an example of a distal end of a lead 700 which may be used with the various embodiments discussed above in relation to FIGS. 1-6. In this example, the lead 700 includes a lead body 706 having an electrode 702 separated by an insulator region 708 from another electrode 704. An electrical conductor 712 is coupled to the electrode 702 and extends proximally back to a proximal end contact as discussed above in relation to the proximal end of the lead examples. Likewise, an electrical conductor 714 is coupled to the electrode 704 and extends proximally back to a proximal end contact. An insulative tip section 710 may be included distally of the electrode 704.

A temperature sensor 718 may be included within one of the electrodes as shown. The temperature sensor 718 may be of various conventional forms such as a thermocouple with an electrical or optical output. A temperature sensor 718 having an electrical output may require a two conductor path back to the probe processing unit and in such a case, multiple insulated conductors may be present within the signal path 716 and multiple electrical contacts may be present on the lead and in the header or separate enclosure at the proximal end to receive the two conduction paths. As another example, the conduction of the body tissue may be utilized as one of the conduction paths.

FIG. 8 shows another example of a distal end of a lead 800 which may be used with the various embodiments discussed above in relation to FIGS. 1-6. In this example, the lead 800 includes a lead body 806 having an electrode 802 separated by an insulator region 808 from another electrode 804. An electrical conductor 812 is coupled to the electrode 802 and extends proximally back to a proximal end contact as discussed above in relation to the proximal end of the lead examples. Likewise, an electrical conductor 814 is coupled to the electrode 804 and extends proximally back to a proximal end contact. An insulative tip section 810 may be included distally of the electrode 804.

A temperature sensor 818 may be included within the insulative region 808 adjacent to the electrodes 802, 804 as shown. The temperature sensor 818 may be of various conventional forms as discussed above in relation to FIG. 7. As discussed above, the temperature sensor 818 having an electrical output may require a two conductor path back to the probe processing unit and in such a case, multiple insulated conductors may be present within the signal path 816 and multiple electrical contacts may be present on the lead and in the header or separate enclosure at the proximal end to receive the two conduction paths. As another example, the conduction of the body tissue may be utilized as one of the conduction paths.

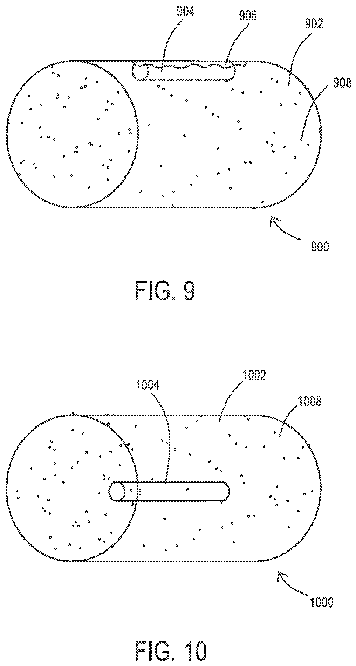

FIG. 9 shows an example 900 of mounting a temperature sensor 904 within an electrode 902 which has the form of a ring. The temperature sensor 904 may be attached to an inside surface of the electrode 902. A medical adhesive 906 may be applied to fix the temperature sensor 904 in position. Furthermore, a conductive epoxy 908 may backfilled within the electrode around the temperature sensor 904.

FIG. 10 shows an example 1000 where a temperature sensor 1004 is suspended within a conductive epoxy filler 1008. The filler 1008 forms a cylinder 1002 that may fit within the insulative region of the lead body between electrodes or within one of the electrodes.

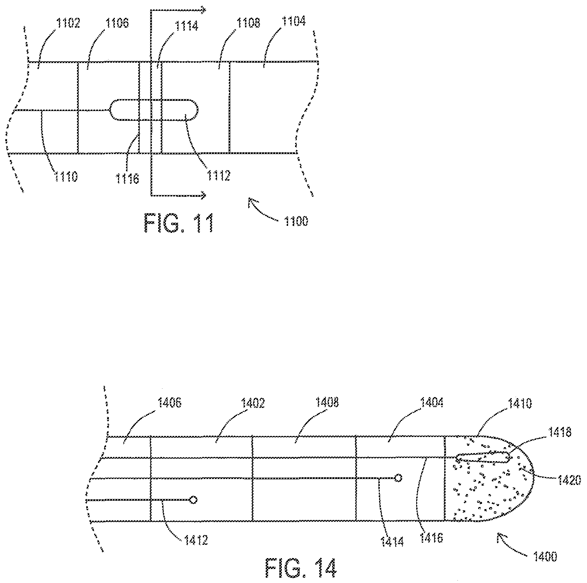

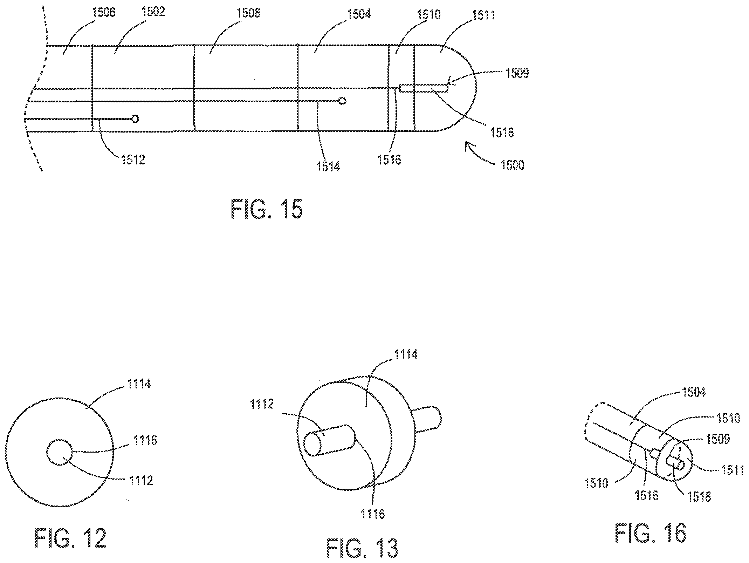

FIG. 11 shows an alternative configuration for including a temperature sensor 1112 adjacent to an electrode 1104. In this example the temperature sensor 1112 is located between an electrode 1102 and the electrode 1104 and is positioned within an aperture 1116 of a conductive ring 1114. This configuration of the temperature sensor 1112 and ring 1114 can be further seen in the lateral cross-sectional view of FIG. 12 and the perspective view of FIG. 13. The conductive ring 1114 is present between insulative portions 1106 and 1108 of the lead 1100 and holds the temperature sensor 1112 in a fixed position. The conductive ring 1114 may be encapsulated within the lead body or may be exposed to the tissue of the patient. The temperature sensor 1112 provides a signal over the signal path 1110 that extends proximally to the proximal end of the lead.

FIG. 14 shows another example of a distal end of a lead 1400 that has electrodes 1402 and 1404 attached to a lead body 1406 and electrical conductors 1412 and 1414 attached to the respective electrodes 1402, 1404. An insulative region 1408 is present between the electrodes 1402, 1404. In this example, the temperature sensor 1418 is located within a tip 1410 of the lead 1400. The tip 1410 may be constructed of an insulative material and then backfilled with a conductive filler material 1420 to fix the temperature sensor 1418 within the tip 1410. The temperature sensor 1418 provides a signal over the signal path 1416 that extends proximally to the proximal end of the lead 1400.

FIG. 15 shows another example of a distal end of a lead 1500 that has electrodes 1502 and 1504 attached to a lead body 1506 and electrical conductors 1512 and 1514 attached to the respective electrodes 1502, 1504. An insulative region 1508 is present between the electrodes 1502, 1504. In this example, the temperature sensor 1518 is located within a tip 1511 of the lead 1500. The tip 1511 is constructed of metal and includes a bore 1509 that the temperature sensor 1518 is tightly positioned within to fix the position of the temperature sensor 1518 within the tip 1511. An additional insulative portion 1510 may be present to separate the metal tip 1511 from the electrode 1504. The configuration of the temperature sensor 1518 and insulative region 1510 can be further seen in the perspective view of FIG. 16. The temperature sensor 1518 provides a signal over the signal path 1516 that extends proximally to the proximal end of the lead 1500.

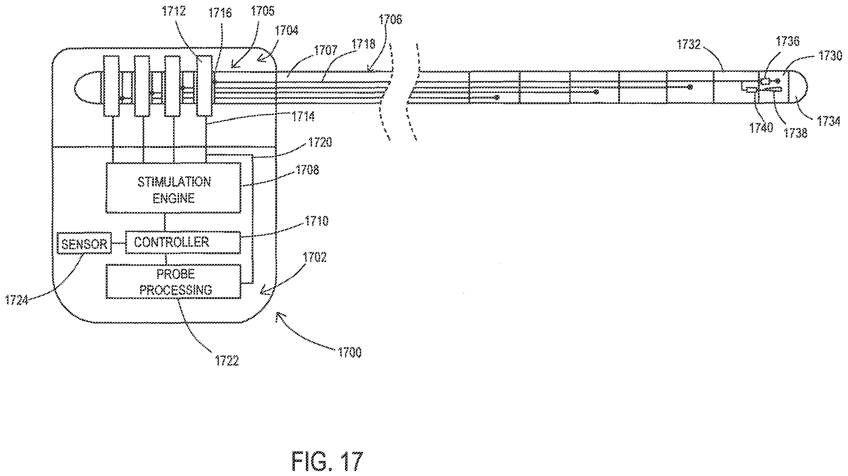

FIG. 17 an embodiment of an implantable medical system 1700 where an IMD 1702 includes a header portion 1704 that includes a first bore 1705 where a proximal end of a lead 1706 is inserted. The proximal end includes an electrical contact 1716 that provides the multiple functions of carrying stimulation or physiological sensing signals and also carrying temperature sensor signals. The electrical contact 1716 couples to an electrical connector 1712 of the header portion 1704. An electrical conductor 1718 that passes through the lead body 1707 to a temperature sensor 1738 and to an electrode 1730 and is electrically coupled to the electrical contact 1716. Likewise, an electrical feedthrough conductor 1714 passes the stimulation signals and electrical signals of the temperature sensor 1738 from the electrical connector 1712 through a conventional feedthrough hermetic seal. Within the IMD 1702, the signal path branches to both a stimulation engine 1708 though portion 1721 and to a probe processing unit 1722 through portion 1720.

The distal end of the lead 1706 includes a tip 1734 and one or more electrodes 1730. While this example shows the temperature sensor 1738 within the electrode 1730, it will be appreciated that any of the temperature sensor mounting methods discussed above may be utilized such as including the temperature sensor in the insulative region 1732 adjacent the electrode 1730 or within the tip 1734.

In order to isolate the temperature sensor 1738 from stimulation or sensed physiological signals during normal operation outside of an MRI scan, a magnetically sensitive switch 1740 configured to close when in the presence of a strong magnetic field from an MRI is in series with the temperature sensor 1738 to isolate the temperature sensor. Likewise, to isolate the electrode 1730 from temperature sensor signals, a second magnetically sensitive switch 1736 configured to open when in the presence of a strong magnetic field from an MRI may be in series with the electrode 1730 to isolate the electrode 1730. An example of the magnetically sensitive switch may be found in U.S. Application No. 61/981,768.

Returning to the IMD 1702, the probe processing unit 1722 may be a conventional temperature sensing unit but in this case has an electrical connection suitable for receiving the electrical signals produced by the temperature sensor 1738. The probe processing unit 1722 may have an output that is electrically coupled to the stimulation engine 1708 and/or to a controller 1710 of the IMD 1702. The probe processing unit 1722 may then output a signal representative of the measured temperature to the controller 1710, and the controller 1710 may then compare the temperature to a threshold and take further action such as causing the stimulation engine 1708 or other circuit to shunt any energy to the device case or other heat sink, or to telemeter an alarm or machine instruction to an external device to cause the MRI scan to be altered. Alternatively, the probe processing unit 1722 may perform additional logic such as comparing the temperature to a threshold and then sending an interrupt signal to the stimulation engine 1722 or other circuit to cause further action to be taken such as operating a switch to shunt the energy away.

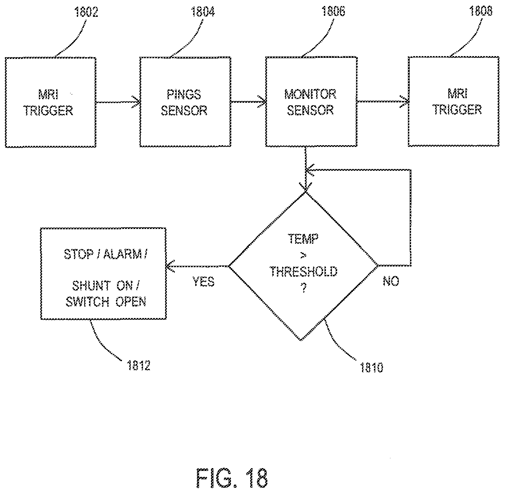

FIG. 18 shows a set of operations that may be performed by a controller of an IMD as introduced for the various embodiments above where a temperature sensor with a fiber optic signal path or a dedicated electrical path is being used. In this example, the controller receives an MRI trigger at an operation 1802, such as receiving a telemetry signal that instructs the controller to enter an MRI mode of operation and/or by having a sensor like that of FIG. 17 that detects the magnetic field of the MRI. Upon receiving the MRI trigger, the controller then pings the temperature sensor by communicating with the probe processing unit of the temperature sensor to cause the probe processing unit to begin providing a temperature reading at an operation 1804. The controller continues to monitor the sensor at an operation 1806.

While monitoring the temperature readings, the controller detects whether the temperature being read exceeds a temperature threshold at a query operation 1810. This process continues until another MRI trigger is received at an operation 1808, such as receiving a telemetry signal indicating the controller should exit the MRI mode of operation or by a sensor no longer detecting the magnetic field of an MRI. If the controller detects that the temperature does exceed a threshold at the query operation 1810, then the controller initiates an action to limit any further increase in the temperature at an operation 1812.

The actions taken may be of various forms. For example, the controller may telemeter an alarm that an external device may produce to cause an MRI technician to reduce the RF power or to terminate the MRI scan. As another example, the controller may telemeter a machine instruction to the MRI scanner that causes the MRI scanner to reduce the RF power or to terminate the MRI scan. As another example, the controller may trigger a switch in series with the electrodes to transition to an open state to block some RF energy from reaching the electrodes, and/or the controller may trigger a shunt in parallel with the electrodes to become active to divert some RF energy away from the electrodes.

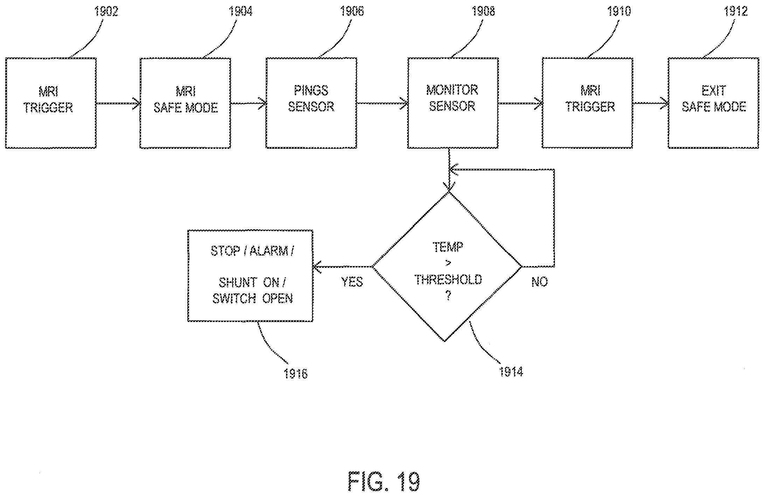

FIG. 19 shows a set of operations that may be performed by a controller of an IMD as introduced for the various embodiments above where a temperature sensor shares an electrical path with an electrode. In this example, the controller receives an MRI trigger at an operation 1902, such as receiving a telemetry signal that instructs the controller to enter an MRI mode of operation and/or by having a sensor like that of FIG. 17 that detects the magnetic field of the MRI. Upon receiving the MRI trigger, the controller then enters an MRI mode of operation where the stimulation path is blocked while the temperature signal path is activated for the shared signal path at an operation 1904. The controller then pings the temperature sensor by communicating with the probe processing unit of the temperature sensor to cause the probe processing unit to begin providing a temperature reading at an operation 1906 by communicating with the temperature sensor over the shared electrical signal path. The controller continues to monitor the sensor at an operation 1908.

While monitoring the temperature readings, the controller detects whether the temperature being read exceeds a temperature threshold at a query operation 1914. This process continues until another MRI trigger is received at an operation 1910, such as receiving a telemetry signal indicating the controller should exit the MRI mode of operation or by a sensor no longer detecting the magnetic field of an MRI. The MRI mode is then exited at operation 1912 where the temperature signal path is then blocked while the stimulation signal path is activated for the shared signal path. If the controller detects that the temperature does exceed a threshold at the query operation 1914, then the controller initiates an action to limit any further increase in the temperature at an operation 1916. The actions taken may include those discussed above in FIG. 18.

Thus, by monitoring the temperature at the electrodes, it can be determined whether operation of the MRI scan may continue as currently configured or whether some action to limit the temperature increase at the electrodes is necessary. Accordingly, the temperature monitoring aids in avoiding risks associated with heating at the electrode to tissue interface due to induced RF energy.

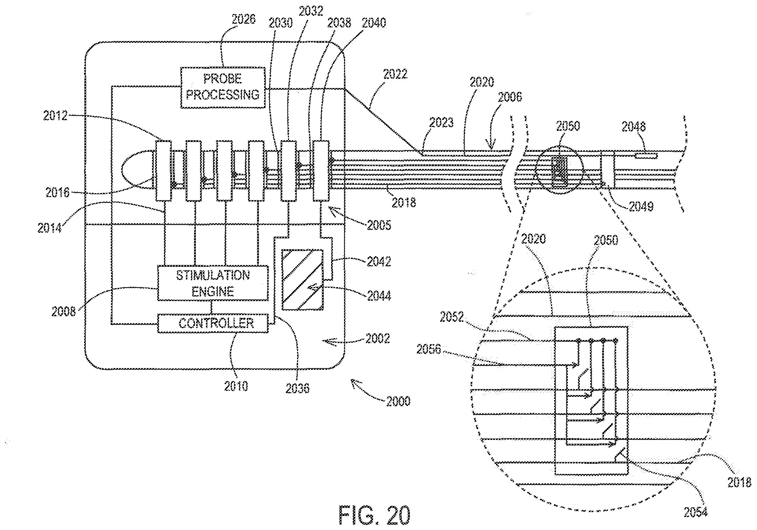

FIG. 20 shows an embodiment of an implantable medical system 2000 where an IMD 2002 includes a header portion that includes a first bore 2005 where a proximal end of a lead 2006 is inserted. Like the embodiment 200 of FIG. 2, the header portion also includes a second bore where a proximal portion 2022 of a fiber optic cable is inserted. The proximal portion 2022 exits from a body of the lead 2006 at an exit point 2023. The remaining portion 2020 extends distally through the body of the lead 2006 to the temperature sensor 2048.

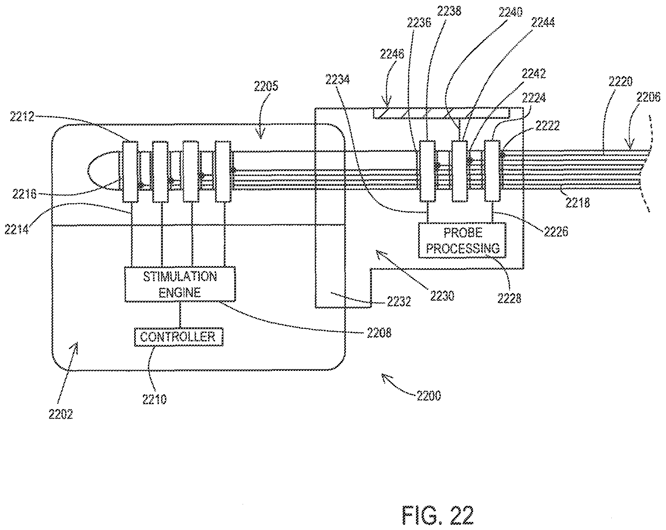

The proximal portion 2022 engages a port of a probe processing unit 2026 that is positioned within the header portion. The probe processing unit 2026 may be a conventional temperature sensing unit with a fiber optic port suitable for receiving the fiber optic cable portion 2022. The probe processing unit 2026 may have an output that is electrically coupled to a stimulation engine 2008 and/or to a controller 2010 of the IMD 2002. The probe processing unit 2026 may then output a signal representative of the measured temperature to a controller 2010, and the controller 2010 may then compare the temperature to a threshold and trigger a shunt switch to shunt energy to a heat sink 2044 at the device 2002, such as to a dedicated heat sink plate or the device casing, in this particular embodiment. The heat sink 2044 may be constructed of various materials, such as a metal capable of conducting the heat to surrounding tissue that are less susceptible to damage than the tissue located at the lead electrode 2049. As discussed below in relation to FIGS. 22-24, the heat sink for the shunt may be located elsewhere. The controller 2010 may additionally or alternatively take additional actions such as those discussed above in relation to FIG. 2.

The lead 2006 includes one or more electrical conductors 2018 that lead to one or more corresponding electrodes 2049 on the distal end and to one or more corresponding electrical contacts 2016 on the proximal end. The header portion includes conventional electrical connectors 2012 that are electrically connected via feed through conductors 2014 passing from the header portion through a conventional feed through hermetic seal to the stimulation engine 2008.

To achieve the shunt, the medical device 2002 of this embodiment includes additional electrical connections to electrically couple to additional electrical connections of the lead 2006. The device includes a connector 2032 that couples to a lead contact 2030. The controller 2010 provides a shunt control output to a conductor 2036 connected to the connector 2032. The contact 2030 receives the signal and a shunt control conductor 2056 carries the shunt control output to a shunt switch block 2050 located in the lead 2006. The shunt switch block 2050 includes a switch 2054 for each stimulation conductor 2018 such that the shunt control output causes the switch 2054 to channel current via a parallel pathway to a shunt conductor 2052 that is also present in the lead 2006. While illustrated as a mechanical switch such as a relay, the switch 2054 may of course be implemented electronically in silicon, for instance as a transistor.

To complete the shunt, the lead 2006 includes a contact 2038 coupled to the shunt conductor 2052. The medical device 2002 includes another additional connector 2040 that electrically couples to the contact 2038. An electrical conductor 2042 then provides an electrical pathway to complete the shunt to the shunt heat sink 2044.

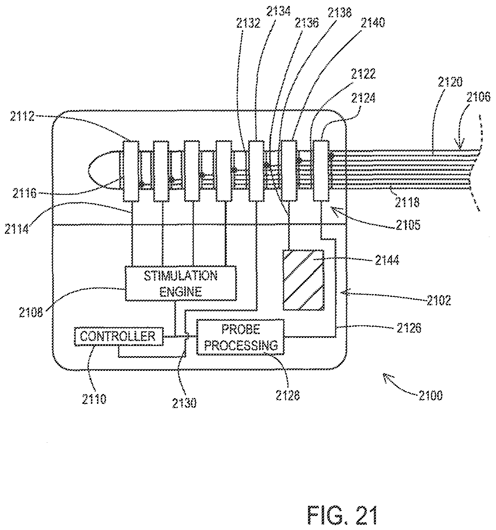

FIG. 21 shows an embodiment of an implantable medical system 2100 where an IMD 2102 includes a header portion that includes a first bore 2105 where a proximal end of a lead 2106 is inserted. The proximal end includes an electrical contact 2122 that is dedicated to temperature sensor signals and couples to a dedicated electrical connector 2124 of the header portion. An electrical conductor 2120 that is dedicated to the temperature sensor located at the distal end of the lead (not shown in this view) passes through the lead body and is electrically coupled to the dedicated electrical contact 2122. Likewise, an electrical feedthrough conductor 2126 passes the electrical signal of the temperature sensor from the electrical connector 2124 through a conventional feedthrough hermetic seal to a probe processing unit 2128 within the IMD 2102.

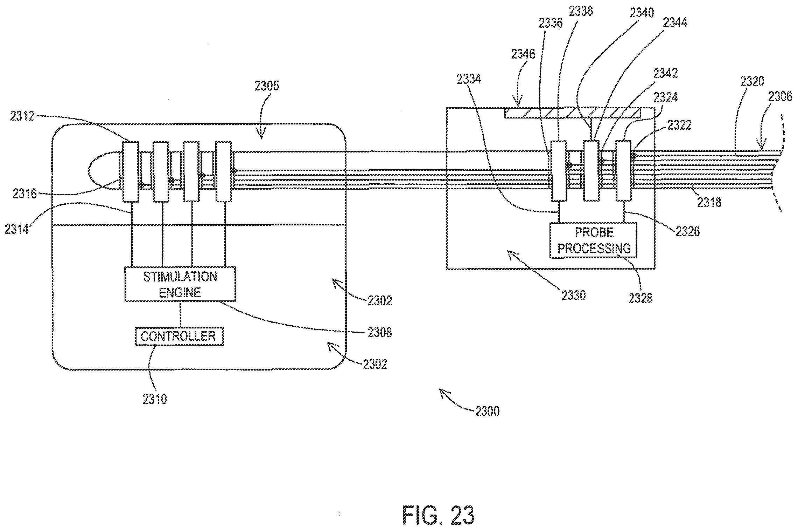

As with FIG. 20, the probe processing unit 2128 may be a conventional temperature sensing unit but in this case has an electrical connection suitable for receiving the electrical signals produced by the temperature sensor connected to the electrical conductor 2120. The probe processing unit 2128 may have an output that is electrically coupled to a stimulation engine 2108 and/or to a controller 2110 of the IMD 2102. The probe processing unit 2128 may then output a signal representative of the measured temperature to the controller 2110, and the controller 2110 may then compare the temperature to a threshold and trigger a shunt switch to shunt energy to a heat sink 2144 at the device 2002, such as to a dedicated heat sink plate or the device casing, in this particular embodiment. The heat sink 2144 may be constructed of materials such as those discussed above in relation to FIG. 20. As discussed below in relation to FIGS. 23-24, the heat sink for the shunt may be located elsewhere. The controller 2010 may additionally or alternatively take additional actions such as those discussed above in relation to FIG. 3.

The lead 2106 also includes one or more electrical conductors 2118 that lead to one or more corresponding electrodes on the distal end (not shown in this view) and to one or more corresponding electrical contacts 2116 on the proximal end. The header portion includes conventional electrical connectors 2112 that are electrically connected via feed through conductors 2114 passing from the header portion through a conventional feed through hermetic seal to the stimulation engine 2108.

To achieve the shunt, the lead 2106 includes the same portion shown in FIG. 20 that includes the shunt switch block 2050, shunt control conductor 2056, and shunt conductor 2052. Also, the medical device 2102 of this embodiment includes additional electrical connections to electrically couple to additional electrical connections of the lead 2106 that are coupled to the conductors 2052, 2056. The device includes a connector 2134 that couples to a lead contact 2132. The controller 2110 provides a shunt control output to a conductor 2130 connected to the connector 2134. The contact 2132 receives the signal and the shunt control conductor 2056 (as in FIG. 20) carries the shunt control output to the shunt switch block 2050 (as in FIG. 20) located in the lead 2106. The shunt switch block 2050 includes the switch 2054 for each stimulation conductor 2118 such that the shunt control output causes the switch 2054 to channel current via a parallel pathway to the shunt conductor 2052 (as in FIG. 20) that is also present in the lead 2106.

To complete the shunt, the lead 2106 includes a contact 2136 coupled to the shunt conductor 2052. The medical device 2002 includes another additional connector 2138 that electrically couples to the contact 2136. An electrical conductor 2136 then provides an electrical pathway to complete the shunt to the shunt heat sink 2144.

FIG. 22 shows an embodiment of an implantable medical system 2200 where a separate probe processing enclosure 2230 is attached to the IMD 2202. The probe processing enclosure 2230 may include various mounting structures 2232 such as a flange that compresses against the IMD 2202 to fix the enclosure 2230 in place. The enclosure 2230 includes a bore where a proximal end of an implantable medical lead 2206 passes entirely through the enclosure 2230 in order to enter a bore 2205 of a header portion of the IMD 2202.

In this example, the lead 2206 includes an electrical contact 2222 that is electrically coupled to a dedicated electrical conductor 2220 within a lead body of the lead 2206. The electrical conductor 2220 extends distally to a temperature sensor at a distal end of the lead 2206 (not shown in this view). The electrical contact 2222 electrically couples to an electrical connector 2224 within the bore of the enclosure 2230.

A probe processing unit 2228 is within the enclosure 2230 and therefore separate from the IMD 2202. In this example, an electrical conductor 2226 electrically couples the probe processing unit 2228 to the electrical connector 2224. The conductor 2226 may pass the electrical signal of the temperature sensor from the electrical connector 2224 through a conventional feedthrough hermetic seal of the enclosure 2230 to the probe processing unit 2228.

While this example shows an electrical conductor 2220 and an electrical signal path to the probe processing unit 2228, it will be appreciated that a fiber cable could be used with the enclosure 2230 and separate probe processing unit. For example, a fiber optic cable within the lead body may exit the lead body distally of the enclosure 2230 and then be received within a bore of the enclosure 2230, in a similar fashion to the manner in which the header portion receives the fiber cable portion 222 in a bore 205 in FIG. 2.

As with the prior examples, the probe processing unit 2228 may be a conventional temperature sensing unit. The probe processing unit 2228 may either be configured to receive an electrical signal via the conductor 2226 or receive a fiber cable in the alternative. The probe processing unit 2228 may have a telemetry circuit to allow the probe processing unit 2228 to provide the temperature information to the controller 2210. The probe processing unit 2228 may also perform additional logic such as comparing the temperature to a threshold and then triggering a shunt switch to shunt the energy to a heat sink 2246 at the probe processing unit, such as to a dedicated heat sink plate or the enclosure 2230, in this particular embodiment. The heat sink 2246 may be constructed of materials like those discussed above for FIG. 20.

In the example shown, the probe processing unit may implement the shunt by including additional electrical connections to electrically couple to additional electrical connections of the lead 2206. The probe processing unit includes a connector 2238 that couples to a lead contact 2236. The probe processing unit 2228 provides a shunt control output to a conductor 2234 connected to the connector 2238. The contact 2236 receives the signal and the shunt control conductor 2056 (as in FIG. 20) carries the shunt control output to the shunt switch block 2050 (as in FIG. 20) located in the lead 2206. The shunt switch block 2050 includes the switch 2054 for each stimulation conductor 2218 such that the shunt control output causes the switch 2054 to channel current via a parallel pathway to the shunt conductor 2052 (as in FIG. 20) that is also present in the lead 2206.

To complete the shunt, the lead 2206 includes a contact 2242 coupled to the shunt conductor 2052. The probe processing unit includes another additional connector 2244 that electrically couples to the contact 2242. An electrical conductor 2240 then provides an electrical pathway to complete the shunt to the shunt heat sink 2246.

The lead 2206 also includes one or more electrical conductors 2218 that lead to one or more corresponding electrodes on the distal end (not shown in this view) and to one or more corresponding electrical contacts 2216 on the proximal end. The header portion includes conventional electrical connectors 2212 that are electrically connected via feed through conductors 2214 passing from the header portion through a conventional feed through hermetic seal to the stimulation engine 2208 which is being controlled by a controller 2210.

FIG. 23 shows an embodiment of an implantable medical system 2300 where a separate probe processing enclosure 2330 is attached to a lead 2306. The probe processing enclosure 2330 may include mounting structures such as an interference fit like that shown in the lateral cross-sectional view of FIG. 6 that compresses against the lead 2306 to fix the enclosure 2330 in place. The enclosure 2330 includes a bore where a proximal end of the implantable medical lead 2306 passes entirely through the enclosure 2330 in order to enter a bore 2305of a header portion of the IMD 2302.

In this example, the lead 2306 includes an electrical contact 2322 that is electrically coupled to a dedicated electrical conductor 2320 within a lead body of the lead 2306. The electrical conductor 2320 extends distally to a temperature sensor at a distal end of the lead 2306 (not shown in this view). The electrical contact 2322 electrically couples to an electrical connector 2324 within the bore of the enclosure 2330.

A probe processing unit 2328 is within the enclosure 2330 and therefore separate from the IMD 2302. In this example, an electrical conductor 2326 electrically couples the probe processing unit 2328 to the electrical connector 2324. The conductor 2326 may pass the electrical signal of the temperature sensor from the electrical connector 2324 through a conventional feedthrough hermetic seal of the enclosure 2330 to the probe processing unit 2328.

While this example shows an electrical conductor 2320 and an electrical signal path to the probe processing unit 2328, it will be appreciated that a fiber cable could be used with the enclosure 2330 and separate probe processing unit. For example, a fiber optic cable within the lead body may exit the lead body distally of the enclosure 2330 and then be received within a bore of the enclosure 2330, in a similar fashion to the manner in which the header portion receives the fiber cable portion 222 in a bore 205 in FIG. 2.

As with the prior examples, the probe processing unit 2328 may be a conventional temperature sensing unit. The probe processing unit 2328 may either be configured to receive an electrical signal via the conductor 2326 or receive a fiber cable in the alternative. The probe processing unit 2328 may have telemetry to communicate with the controller 2310 as described above for the probe processing unit of FIG. 4. The probe processing unit 2328 may also perform additional logic such as comparing the temperature to a threshold and then trigger a shunt switch to shunt the energy to a heat sink 2346 at the probe processing unit, such as to a dedicated heat sink plate or the enclosure 2330, in this particular embodiment. The heat sink 2346 may be constructed of materials like those discussed above for FIG. 20.

In the example shown, the probe processing unit may implement the shunt by including additional electrical connections to electrically couple to additional electrical connections of the lead 2306. The probe processing unit includes a connector 2338 that couples to a lead contact 2336. The probe processing unit 2328 provides a shunt control output to a conductor 2334 connected to the connector 2338. The contact 2336 receives the signal and the shunt control conductor 2056 (as in FIG. 20) carries the shunt control output to the shunt switch block 2050 (as in FIG. 20) located in the lead 2306. The shunt switch block 2050 includes the switch 2054 for each stimulation conductor 2318 such that the shunt control output causes the switch 2054 to channel current via a parallel pathway to a shunt conductor 2052 (as in FIG. 20) that is also present in the lead 2306.

To complete the shunt, the lead 2306 includes a contact 2342 coupled to the shunt conductor 2052. The probe processing unit includes another additional connector 2344 that electrically couples to the contact 2342. An electrical conductor 2340 then provides an electrical pathway to complete the shunt to the shunt heat sink 2346.

The lead 2306 also includes one or more electrical conductors 2318 that lead to one or more corresponding electrodes on the distal end (not shown in this view) and to one or more corresponding electrical contacts 2316 on the proximal end. The header portion includes conventional electrical connectors 2312 that are electrically connected via feed through conductors 2314 passing from the header portion through a conventional feed through hermetic seal to the stimulation engine 2308 which is being controlled by a controller 2310.

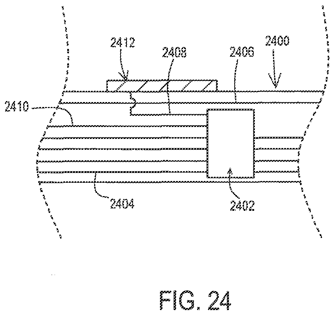

FIG. 24 shows an example of a medical lead 2400 that includes a heat sink 2412 located on the lead body that works in conjunction with a shunt switch block 2402 located within the body of the lead 2400. The switch block 2402 may be configured in the same manner as the switch block 2050 of FIG. 20, receiving stimulation conductors 2404, shunt control conductor 2410, and shunt conductor 2408. When the shunt is active due to an issue with temperature detected by a controller of an IMD connected to the lead 2400 or a probe processing unit receiving the lead 2400, the parallel conduction path from the stimulation conductors 2404 to the shunt conductor 2408 is provided and channels excess current to the heat sink 2412. A temperature conductor 2406 is present to carry the temperature signals from a temperature sensor located within the lead to the controller or probe processing unit.

The heat sink 2412 may be constructed in various manners. For instance, the heat sink 2412 may be a metal ring located about the lead body.

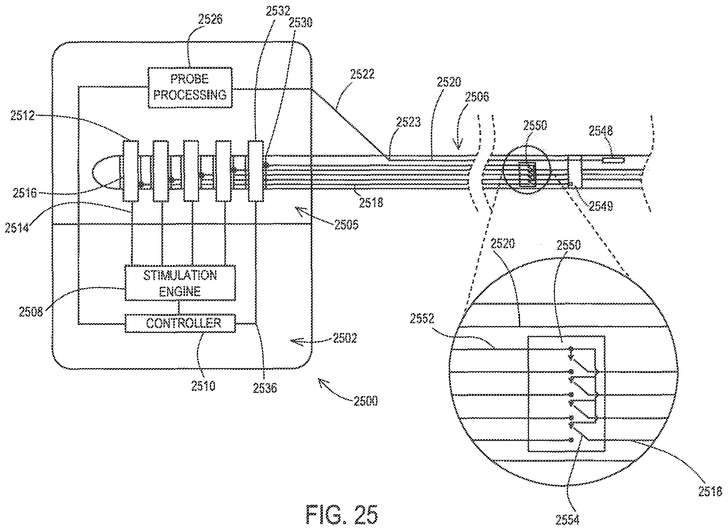

FIG. 25 shows an embodiment of an implantable medical system 2500 where an IMD 2502 includes a header portion that includes a first bore 2505 where a proximal end of a lead 2506 is inserted. Like the embodiment 200 of FIG. 2, the header portion also includes a second bore where a proximal portion 2522 of a fiber optic cable is inserted. The proximal portion 2522 exits from a body of the lead 2506 at an exit point 2523. The remaining portion 2520 extends distally through the body of the lead 2506 to the temperature sensor 2548.

The proximal portion 2522 engages a port of a probe processing unit 2526 that is positioned within the header portion. The probe processing unit 2526 may be a conventional temperature sensing unit with a fiber optic port suitable for receiving the fiber optic cable portion 2522. The probe processing unit 2526 may have an output that is electrically coupled to a stimulation engine 2508 and/or to a controller 2510 of the IMD 2502. The probe processing unit 2526 may then output a signal representative of the measured temperature to a controller 2510, and the controller 2510 may then compare the temperature to a threshold and trigger a series switch to block at least to some degree the energy from being conducted electrically within the lead 2506. The controller 2510 may additionally or alternatively take additional actions such as those discussed above in relation to FIG. 2.

The lead 2506 includes one or more electrical conductors 2518 that lead to one or more corresponding electrodes 2549 on the distal end and to one or more corresponding electrical contacts 2516 on the proximal end. The header portion includes conventional electrical connectors 2512 that are electrically connected via feed through conductors 2514 passing from the header portion through a conventional feed through hermetic seal to the stimulation engine 2508.

To achieve the blocking of energy conduction, the medical device 2502 of this embodiment includes additional electrical connections to electrically couple to additional electrical connections of the lead 2506. The device includes a connector 2532 that couples to a lead contact 2530. The controller 2510 provides a series control output to a conductor 2536 connected to the connector 2532. The contact 2530 receives the signal and a series control conductor 2552 carries the series control output to a series switch block 2550 located in the lead 2506. The series switch block 2550 includes a switch 2554 for each stimulation conductor 2518 such that the series control output causes the switch 2554 to create a large RF frequency impedance such as an open circuit condition with minimal capacitance in the lead 2506. While illustrated as a mechanical switch such as a relay, the switch 2554 may of course be implemented electronically in silicon, for instance as a transistor.

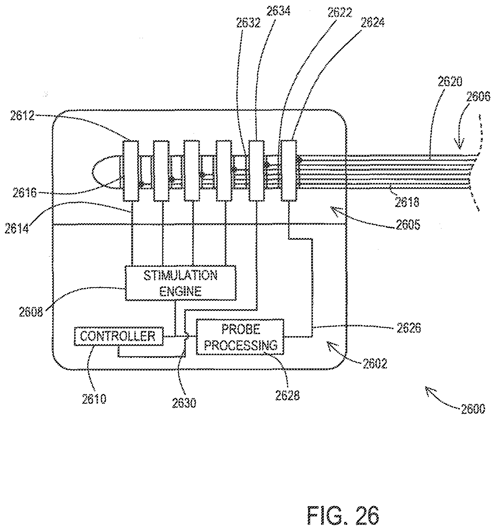

FIG. 26 shows an embodiment of an implantable medical system 2600 where an IMD 2602 includes a header portion that includes a first bore 2605 where a proximal end of a lead 2606 is inserted. The proximal end includes an electrical contact 2622 that is dedicated to temperature sensor signals and couples to a dedicated electrical connector 2624 of the header portion. An electrical conductor 2620 that is dedicated to the temperature sensor located at the distal end of the lead (not shown in this view) passes through the lead body and is electrically coupled to the dedicated electrical contact 2622. Likewise, an electrical feedthrough conductor 2626 passes the electrical signal of the temperature sensor from the electrical connector 2624 through a conventional feedthrough hermetic seal to a probe processing unit 2628 within the IMD 2602.

As with FIG. 25, the probe processing unit 2628 may be a conventional temperature sensing unit but in this case has an electrical connection suitable for receiving the electrical signals produced by the temperature sensor connected to the electrical conductor 2620. The probe processing unit 2628 may have an output that is electrically coupled to a stimulation engine 2608 and/or to a controller 2610 of the IMD 2602. The probe processing unit 2628 may then output a signal representative of the measured temperature to the controller 2610, and the controller 2610 may then compare the temperature to a threshold and trigger a series switch to block at least to some degree the energy from being conducted electrically within the lead 2606. The controller 2610 may additionally or alternatively take additional actions such as those discussed above in relation to FIG. 3.

The lead 2606 also includes one or more electrical conductors 2618 that lead to one or more corresponding electrodes on the distal end (not shown in this view) and to one or more corresponding electrical contacts 2616 on the proximal end. The header portion includes conventional electrical connectors 2612 that are electrically connected via feed through conductors 2614 passing from the header portion through a conventional feed through hermetic seal to the stimulation engine 2608.

To achieve the blocking of energy conduction, the medical device 2602 of this embodiment includes additional electrical connections to electrically couple to additional electrical connections of the lead 2606. The device includes a connector 2634 that couples to a lead contact 2632. The controller 2610 provides a series control output to a conductor 2630 connected to the connector 2634. The contact 2632 receives the signal and a series control conductor 2552 (as in FIG. 25) carries the series control output to the series switch block 2550 (as in FIG. 25) located in the lead 2606. The series switch block 2550 includes the switch 2554 for each stimulation conductor 2618 such that the series control output causes the switch 2554 to create a large RF frequency impedance such as an open circuit condition with minimal capacitance in the lead 2606. While illustrated as a mechanical switch such as a relay, the switch 2654 may of course be implemented electronically in silicon, for instance as a transistor.

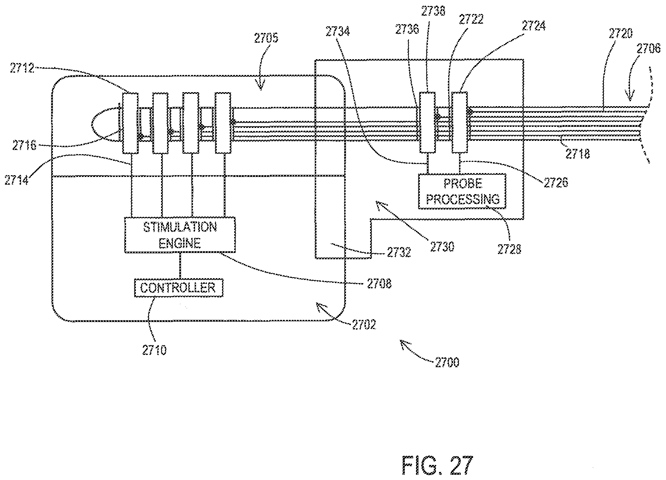

FIG. 27 shows an embodiment of an implantable medical system 2700 where a separate probe processing enclosure 2730 is attached to the IMD 2702. The probe processing enclosure 2730 may include various mounting structures 2732 such as a flange that compresses against the IMD 2702 to fix the enclosure 2730 in place. The enclosure 2730 includes a bore where a proximal end of an implantable medical lead 2706 passes entirely through the enclosure 2730 in order to enter a bore 2705 of a header portion of the IMD 2702.

In this example, the lead 2706 includes an electrical contact 2722 that is electrically coupled to a dedicated electrical conductor 2720 within a lead body of the lead 2706. The electrical conductor 2720 extends distally to a temperature sensor at a distal end of the lead 2706 (not shown in this view). The electrical contact 2722 electrically couples to an electrical connector 2724 within the bore of the enclosure 2730.

A probe processing unit 2728 is within the enclosure 2730 and therefore separate from the IMD 2702. In this example, an electrical conductor 2726 electrically couples the probe processing unit 2728 to the electrical connector 2724. The conductor 2726 may pass the electrical signal of the temperature sensor from the electrical connector 2724 through a conventional feedthrough hermetic seal of the enclosure 2730 to the probe processing unit 2728.

While this example shows an electrical conductor 2720 and an electrical signal path to the probe processing unit 2728, it will be appreciated that a fiber cable could be used with the enclosure 2730 and separate probe processing unit. For example, a fiber optic cable within the lead body may exit the lead body distally of the enclosure 2730 and then be received within a bore of the enclosure 2730, in a similar fashion to the manner in which the header portion receives the fiber cable portion 222 in a bore 205 in FIG. 2.

As with the prior examples, the probe processing unit 2728 may be a conventional temperature sensing unit. The probe processing unit 2728 may either be configured to receive an electrical signal via the conductor 2726 or receive a fiber cable in the alternative. The probe processing unit 2728 may have a telemetry circuit to allow the probe processing unit 2728 to provide the temperature information to the controller 2710. The probe processing unit 2728 may also perform additional logic such as comparing the temperature to a threshold and then triggering a series switch to block at least to some degree the energy from being conducted electrically within the lead 2706. The controller 2710 may additionally or alternatively take additional actions such as those discussed above in relation to FIG. 2.

The lead 2706 also includes one or more electrical conductors 2718 that lead to one or more corresponding electrodes on the distal end (not shown in this view) and to one or more corresponding electrical contacts 2716 on the proximal end. The header portion includes conventional electrical connectors 2712 that are electrically connected via feed through conductors 2714 passing from the header portion through a conventional feed through hermetic seal to the stimulation engine 2708 which is being controlled by a controller 2710.

To achieve the blocking of energy conduction, the probe processing unit of this embodiment includes additional electrical connections to electrically couple to additional electrical connections of the lead 2706. The probe processing unit includes a connector 2736 that couples to a lead contact 2734. The probe processing unit 2728 provides a series control output to a conductor 2734 connected to the connector 2736. The contact 2734 receives the signal and a series control conductor 2552 (as in FIG. 25) carries the series control output to a series switch block 2550 (as in FIG. 25) located in the lead 2706. The series switch block 2550 includes the switch 2554 for each stimulation conductor 2718 such that the series control output causes the switch 2554 to create a large RF frequency impedance such as an open circuit condition with minimal capacitance in the lead 2706. While illustrated as a mechanical switch such as a relay, the switch 2554 may of course be implemented electronically in silicon, for instance as a transistor.

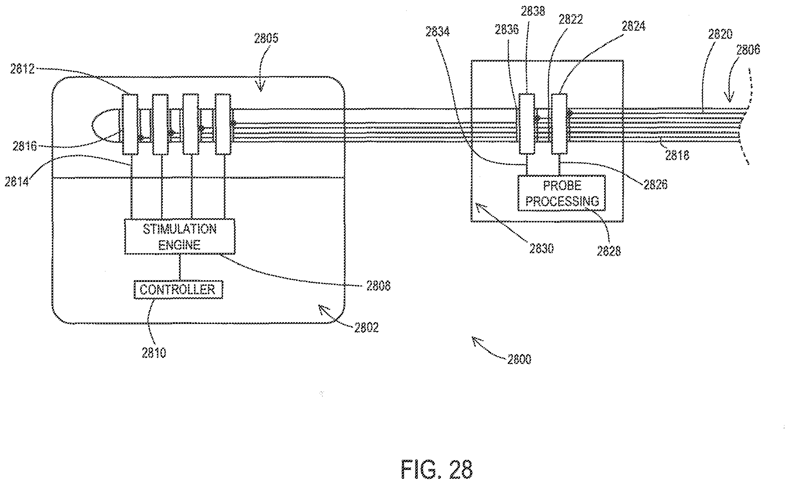

FIG. 28 shows an embodiment of an implantable medical system 2800 where a separate probe processing enclosure 2830 is attached to a lead 2806. The probe processing enclosure 2830 may include mounting structures such as an interference fit like that shown in the lateral cross-sectional view of FIG. 6 that compresses against the lead 2806 to fix the enclosure 2830 in place. The enclosure 2830 includes a bore where a proximal end of the implantable medical lead 2806 passes entirely through the enclosure 2830 in order to enter a bore 2805of a header portion of the IMD 2802.

In this example, the lead 2806 includes an electrical contact 2822 that is electrically coupled to a dedicated electrical conductor 2820 within a lead body of the lead 2806. The electrical conductor 2820 extends distally to a temperature sensor at a distal end of the lead 2806 (not shown in this view). The electrical contact 2822 electrically couples to an electrical connector 2824 within the bore of the enclosure 2830.

A probe processing unit 2828 is within the enclosure 2830 and therefore separate from the IMD 2802. In this example, an electrical conductor 2826 electrically couples the probe processing unit 2828 to the electrical connector 2824. The conductor 2826 may pass the electrical signal of the temperature sensor from the electrical connector 2824 through a conventional feedthrough hermetic seal of the enclosure 2830 to the probe processing unit 2828.

While this example shows an electrical conductor 2820 and an electrical signal path to the probe processing unit 2828, it will be appreciated that a fiber cable could be used with the enclosure 2830 and separate probe processing unit. For example, a fiber optic cable within the lead body may exit the lead body distally of the enclosure 2830 and then be received within a bore of the enclosure 2830, in a similar fashion to the manner in which the header portion receives the fiber cable portion 222 in a bore 205 in FIG. 2.

As with the prior examples, the probe processing unit 2828 may be a conventional temperature sensing unit. The probe processing unit 2828 may either be configured to receive an electrical signal via the conductor 2826 or receive a fiber cable in the alternative. The probe processing unit 2828 may have telemetry to communicate with the controller 2810 as described above for the probe processing unit of FIG. 4. The probe processing unit 2828 may also perform additional logic such as comparing the temperature to a threshold and then triggering a series switch to block at least to some degree the energy from being conducted electrically within the lead 2806. The controller 2810 may additionally or alternatively take additional actions such as those discussed above in relation to FIG. 2.

The lead 2806 also includes one or more electrical conductors 2818 that lead to one or more corresponding electrodes on the distal end (not shown in this view) and to one or more corresponding electrical contacts 2816 on the proximal end. The header portion includes conventional electrical connectors 2812 that are electrically connected via feed through conductors 2814 passing from the header portion through a conventional feed through hermetic seal to the stimulation engine 2808 which is being controlled by a controller 2810.

To achieve the blocking of energy conduction, the probe processing unit of this embodiment includes additional electrical connections to electrically couple to additional electrical connections of the lead 2806. The probe processing unit includes a connector 2838 that couples to a lead contact 2836. The probe processing unit 2828 provides a series control output to a conductor 2834 connected to the connector 2838. The contact 2836 receives the signal and a series control conductor 2552 (as in FIG. 25) carries the series control output to the series switch block 2550 (as in FIG. 25) located in the lead 2806. The series switch block 2550 includes the switch 2554 for each stimulation conductor 2818 such that the series control output causes the switch 2554 to create a large RF frequency impedance such as an open circuit condition with minimal capacitance in the lead 2806. While illustrated as a mechanical switch such as a relay, the switch 2554 may of course be implemented electronically in silicon, for instance as a transistor.

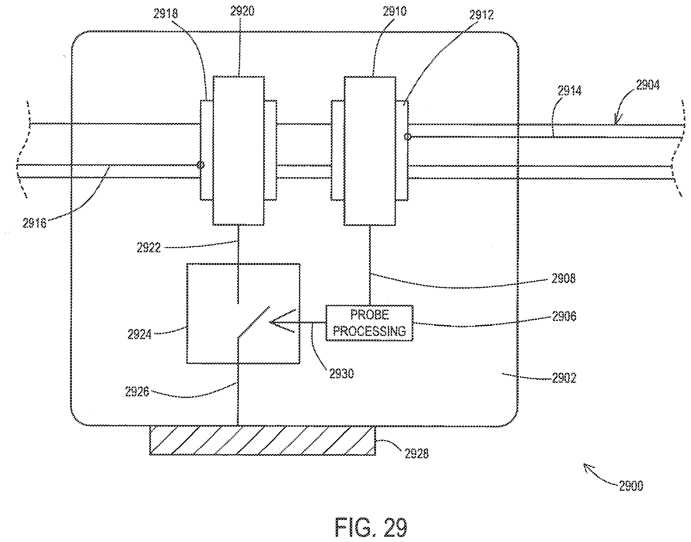

FIG. 29 shows an alternative manner of implementing a shunt for embodiments of a probe processing unit like that of FIGS. 22 and 23. A separate enclosure 2902 from an IMD is coupled to a lead 2904, where the lead 2904 is then coupled to an IMD. The lead 2904 includes a conductor 2914 connected to a temperature sensor (not shown) located in a distal end of the lead 2904. The lead includes a contact 2912 that is coupled to an electrical connector 2910 of the enclosure 2902. A probe processing unit 2906 is coupled to the connector 2910 via a conductor 2908. As an alternative, a fiber optic path may be provided from the temperature sensor to the probe processing unit 2906.

The probe processing unit 2906 compares the temperature to a threshold and then when the temperature is too high may trigger a switch block 2924 via a control connection 2930 to create a parallel path from a conductor 2922 to a conductor 2926. The conductor 2922 is connected to a connector 2920 which is coupled to a contact 2918 of the lead, which is in turn coupled to a stimulation conductor 2916 of the lead 2904. The conductor 2926 is connected to a heat sink 2928. Thus, the switch block 2924 creates a parallel path for energy from the stimulation conductor 2916 to the heat sink 2928.

To the extent there are multiple stimulation conductors in the lead 2904, there may be separate electrical paths for each of those stimulation conductors to separate switches of the switch block 2924. In this manner, the multiple stimulation conductors are not joined as a single electrical node unless the switch block 2924 activates to create the parallel conduction path to the heat sink 2928. The separate electrical paths may be created by having a separate contact on the lead coupled to a separate connector within the enclosure 2902. Alternatively, a segmented contact and a segmented connector could be implemented where each segment corresponds to a separate stimulation conductor 2916.

While embodiments have been particularly shown and described, it will be understood by those skilled in the art that various other changes in the form and details may be made therein without departing from the spirit and scope of the invention.

## Claims

1. A method of reducing heating at an electrode (2049) on a lead (2006, 2106, 2206, 2306, 2406) of an implantable medical system (2000, 2100, 2200, 2300, 2400) during an MRI scan, wherein the lead (2006, 2106, 2206, 2306, 2406) includes one or more stimulation conductors (2404) and a shunt conductor (2052, 2240, 2320, 2408), the method comprising:
monitoring a temperature at the one or more electrodes (2049) during an MRI scan; and
upon detecting that the temperature being monitored exceeds a threshold, then shunting, within the shunt conductor (2052, 2240, 2320, 2408) of the lead (2006, 2106, 2206, 2306, 2406), electrical current from the one or more stimulation conductors (2404) to a heat sink (2044, 2144, 2246, 2346, 2412) externally located on the implantable medical system (2200, 2300, 2400).

2. The method of claim 1, wherein the heat sink (2044, 2144, 2246, 2346, 2412) comprises an external casing of an implantable medical device (2200) coupled to the lead (2006, 2106, 2206, 2306, 2406).

3. The method of claim 1, wherein the heat sink (2044, 2144, 2246, 2346, 2412) comprises an enclosure (2330) of a probe processing unit coupled to an implantable medical device of the implantable medical system or wherein the heat sink (2044, 2144, 2246, 2346, 2412) is positioned on an external surface of a lead body of the lead (2006, 2106, 2206, 2306, 2406).

4. The method of claim 1, wherein monitoring the temperature comprises including a temperature sensor within a lead body of the lead (2006, 2106, 2206, 2306, 2406) and in a position adjacent the electrode (2049).

5. The method of claim 4, wherein including the temperature sensor comprises including a fiber optic cable within the lead body and attached to the temperature sensor such that temperature sensor signals are transferred over the fiber optic cable or wherein including the temperature sensor comprises including an electrical connection between the temperature sensor and an electrical conductor within the lead body.

6. The method of claim 4, wherein including the temperature sensor comprises including an electrical connection between the temperature sensor and an electrical conductor within the lead body and wherein the electrical conductor is electrically coupled to the electrode.

7. The method of claim 6, wherein the electrical conductor extends to a proximal contact on the lead, specifically wherein the proximal contact resides within a bore of a header of an implantable medical device when the lead is fully inserted into the bore.

8. A method of reducing heating at an electrode on a lead (2506, 2606, 2706, 2806) of an implantable medical system (2500, 2600, 2700, 2800) during an MRI scan, comprising:
monitoring a temperature at the electrode during an MRI scan; and
upon detecting that the temperature being monitored exceeds a threshold, then blocking electrical current within a stimulation conductor (2518, 2618, 2718, 2818) of the lead by creating an open circuit with a control conductor (2552) coupled to a switch (2554) that is connected in series with the stimulation conductor.

9. The method of claim 8, wherein the lead (2506, 2606, 2706, 2806) includes a plurality of stimulation conductors, wherein there is a separate switch that is connected in series with each stimulation conductor of the plurality, and wherein the control conductor is coupled to each of the separate switches.

10. The method of claim 8, wherein the switch (2554) is included within a probe processing unit coupled to the lead (2506, 2606, 2706, 2806) or wherein the switch (2554) is positioned within a lead body of the lead (2506, 2606, 2706, 2806).

11. The method of claim 9, wherein monitoring the temperature comprises including a temperature sensor within a lead body of the lead and in a position adj acent the electrode.

12. The method of claim 11, wherein including the temperature sensor comprises including a fiber optic cable within the lead body and attached to the temperature sensor such that temperature sensor signals are transferred over the fiber optic cable or wherein including the temperature sensor comprises including an electrical connection between the temperature sensor and an electrical conductor within the lead body.

13. The method of claim 11, wherein including the temperature sensor comprises including an electrical connection between the temperature sensor and an electrical conductor within the lead body and wherein the electrical conductor is electrically coupled to the electrode, specifically wherein the electrical conductor extends to a proximal contact on the lead (2506, 2606, 2706, 2806), more specifically, wherein the proximal contact resides within a bore of a header of an implantable medical device when the lead is fully inserted into the bore.

14. An implantable medical system, comprising:
a heat sink (2044, 2144, 2246, 2346, 2412);
an implantable medical device that comprises:
a stimulation engine (2008, 2108, 2208, 2308); and
an electrical connector (2124) that is electrically coupled to an output of the stimulation engine (2008, 2108, 2208, 2308);
an implantable medical lead (2006, 2106, 2206, 2306, 2406) that comprises:
a lead body;
a proximal contact on a proximal end of the lead body, the proximal contact being electrically coupled to the electrical connector (2124);
a distal electrode on a distal end of the lead body;
an electrical conductor (2018, 2118, 2218, 2318) that electrically interconnects the proximal contact to the distal electrode, with the proximal contact electrically coupled to the electrical connector (2124) so as to create a conduction path from the electrical connector (2124) to the distal electrode;
a temperature sensor (2548) within the lead body and adjacent the distal electrode; and
a signal path extending proximally from the temperature sensor (2548) through the lead body;
a switch (2050) having a control input, a first connection electrically connected to the heat sink (2044, 2144, 2246, 2346, 2412), and a second connection electrically connected to the conduction path so as to be in parallel with a portion of the conduction path that extends from the second connection to the distal electrode such that when the switch (2050) is closed the conduction path is shunted to the heat sink (2044, 2144, 2246, 2346, 2412) through the switch (2054); and
a controller (2010, 2110, 2210, 2310) in communication with the control input of the switch (2050) and in communication with the signal path of the temperature sensor (2548) such that the controller receives a signal representative of temperature from the temperature sensor (2548) and closes the switch (2054) when the temperature as represented by the received signal exceeds a threshold.

15. An implantable medical system, comprising:
an implantable medical device that comprises:
a stimulation engine; and
an electrical connector (2736) that is electrically coupled to an output of the stimulation engine; and
an implantable medical lead (2506, 2606, 2706, 2806) that comprises:
a lead body;
a proximal contact on a proximal end of the lead body, the proximal contact being electrically coupled to the electrical connector (2736);
a distal electrode on a distal end of the lead body;
an electrical conductor (2518, 2618, 2718, 2818) that electrically interconnects the proximal contact to the distal electrode, with the proximal contact electrically coupled to the electrical connector so (2736) as to create a conduction path from the electrical connector (2736) to the distal electrode;
a temperature sensor (2548) within the lead body and adjacent the distal electrode; and
a signal path extending proximally from the temperature sensor (2548) through the lead body;
a switch (2554) having a control input and being connected in series in the conduction path such that when the switch (2554) is opened the conduction path is an open circuit at the switch (2554); and
a controller (2510, 2610, 2710, 2810) in communication with the control input of the switch (2554) and in communication with the signal path of the temperature sensor such that the controller (2510, 2610, 2710, 2810) receives a signal representative of temperature from the temperature sensor (2548) and opens the switch (2554) when the temperature as represented by the received signal exceeds a threshold.

## Patentansprüche

1. Verfahren zur Reduzierung der Erwärmung an einer Elektrode (2049) auf einer Leitung (2006, 2106, 2206, 2306, 2406) eines implantierbaren medizinischen Systems (2000, 2100, 2200, 2300, 2400) während eines MRI-Scans, wobei die Leitung (2006, 2106, 2206, 2306, 2406) einen oder mehrere Stimulationsleiter (2404) und einen Nebenschlussleiter (2052, 2240, 2320, 2408) umfasst, wobei das Verfahren umfasst:
Überwachen einer Temperatur an der einen oder den mehreren Elektroden (2049) während eines MRI-Scans; und
dann, wenn erkannt wird, dass die Temperatur, die überwacht wird, eine Schwelle überschreitet, Nebenschließen von elektrischem Strom von dem einen oder den mehreren Stimulationsleitern (2404) innerhalb des Nebenschlussleiters (2052, 2240, 2320, 2408) der Leitung (2006, 2106, 2206, 2306, 2406) zu einer Wärmesenke (2044, 2144, 2246, 2346, 2412), die sich außen auf dem implantierbaren medizinischen System (2200, 2300, 2400) befindet.

2. Verfahren nach Anspruch 1, wobei die Wärmesenke (2044, 2144, 2246, 2346, 2412) ein Außengehäuse einer implantierbaren medizinischen Vorrichtung (2200) umfasst, die mit der Leitung (2006, 2106, 2206, 2306, 2406) gekoppelt ist.

3. Verfahren nach Anspruch 1, wobei die Wärmesenke (2044, 2144, 2246, 2346, 2412) ein Gehäuse (2330) einer Sondenverarbeitungseinheit umfasst, die mit einer implantierbaren medizinischen Vorrichtung des implantierbaren medizinischen Systems gekoppelt ist, oder wobei die Wärmesenke (2044, 2144, 2246, 2346, 2412) auf einer Außenfläche eines Leitungskörpers der Leitung (2006, 2106, 2206, 2306, 2406) positioniert ist.

4. Verfahren nach Anspruch 1, wobei das Überwachen der Temperatur ein Aufnehmen eines Temperatursensors innerhalb eines Leitungskörpers der Leitung (2006, 2106, 2206, 2306, 2406) und in einer Position benachbart zur Elektrode (2049) umfasst.

5. Verfahren nach Anspruch 4, wobei das Aufnehmen des Temperatursensors ein Aufnehmen eines Glasfaserkabels innerhalb des Leitungskörpers und angeschlossen an den Temperatursensor umfasst, sodass Temperatursensorsignale über das Glasfaserkabel übertragen werden, oder wobei das Aufnehmen des Temperatursensors ein Aufnehmen einer elektrischen Verbindung zwischen dem Temperatursensor und einem elektrischen Leiter innerhalb des Leitungskörpers umfasst.

6. Verfahren nach Anspruch 4, wobei das Aufnehmen des Temperatursensors ein Aufnehmen einer elektrischen Verbindung zwischen dem Temperatursensor und einem elektrischen Leiter innerhalb des Leitungskörpers umfasst und wobei der elektrische Leiter elektrisch mit der Elektrode gekoppelt ist.

7. Verfahren nach Anspruch 6, wobei sich der elektrische Leiter zu einem proximalen Kontakt auf der Leitung erstreckt, wobei sich der proximale Kontakt insbesondere innerhalb einer Bohrung eines Kopfstücks einer implantierbaren medizinischen Vorrichtung befindet, wenn die Leitung vollständig in die Bohrung eingeführt ist.

8. Verfahren zur Reduzierung von Erwärmung an einer Elektrode auf einer Leitung (2506, 2606, 2706, 2806) eines implantierbaren medizinischen Systems (2500, 2600, 2700, 2800) während eines MRI-Scans, umfassend:
Überwachen einer Temperatur an der Elektrode (2049) während eines MRI-Scans; und
dann, wenn erkannt wird, dass die Temperatur, die überwacht wird, eine Schwelle überschreitet, Sperren von elektrischem Strom innerhalb eines Stimulationsleiters (2518, 2618, 2718, 2818) der Leitung durch Bilden eines offenen Kreises mit einem Steuerleiter (2552), der mit einem Schalter (2554) gekoppelt ist, der mit dem Stimulationsleiter in Reihe geschaltet ist.

9. Verfahren nach Anspruch 8, wobei die Leitung (2506, 2606, 2706, 2806) eine Mehrzahl von Stimulationsleitern umfasst, wobei es einen separaten Schalter gibt, der mit jedem Stimulationsleiter der Mehrzahl in Reihe geschaltet ist, und wobei der Steuerleiter mit jedem der separaten Schalter gekoppelt ist.

10. Verfahren nach Anspruch 1, wobei der Schalter (2554) innerhalb einer Sondenverarbeitungseinheit aufgenommen wird, die mit der Leitung (2506, 2606, 2706, 2806) gekoppelt ist, oder wobei der Schalter (2554) innerhalb eines Leitungskörpers der Leitung (2506, 2606, 2706, 2806) positioniert wird.

11. Verfahren nach Anspruch 9, wobei das Überwachen der Temperatur ein Aufnehmen eines Temperatursensors innerhalb eines Leitungskörpers der Leitung und in einer Position benachbart zur Elektrode umfasst.

12. Verfahren nach Anspruch 11, wobei das Aufnehmen des Temperatursensors ein Aufnehmen eines Glasfaserkabels innerhalb des Leitungskörpers und angeschlossen an den Temperatursensor umfasst, sodass Temperatursensorsignale über das Glasfaserkabel übertragen werden, oder wobei das Aufnehmen des Temperatursensors ein Aufnehmen einer elektrischen Verbindung zwischen dem Temperatursensor und einem elektrischen Leiter innerhalb des Leitungskörpers umfasst.

13. Verfahren nach Anspruch 11, wobei das Aufnehmen des Temperatursensors ein Aufnehmen einer elektrischen Verbindung zwischen dem Temperatursensor und einem elektrischen Leiter innerhalb des Leitungskörpers umfasst und wobei der elektrische Leiter elektrisch mit der Elektrode gekoppelt wird, wobei sich der elektrische Leiter insbesondere zu einem proximalen Kontakt auf der Leitung (2506, 2606, 2706, 2806) erstreckt, wobei sich der proximale Kontakt genauer gesagt innerhalb einer Bohrung eines Kopfstücks einer implantierbaren medizinischen Vorrichtung befindet, wenn die Leitung vollständig in die Bohrung eingeführt ist.

14. Implantierbares medizinisches System, umfassend:
eine Wärmsenke (2044, 2144, 2246, 2346, 2412);
eine implantierbare medizinische Vorrichtung, die umfasst:
einen Stimulationsmotor (2008, 2108, 2208, 2308); und
einen elektrischen Verbinder (2124), der mit einem Ausgang des Stimulationsmotors (2008, 2108, 2208, 2308) elektrisch gekoppelt ist;
eine implantierbare medizinische Leitung (2006, 2106, 2206, 2306, 2406), die umfasst:
einen Leitungskörper;
einen proximalen Kontakt an einem proximalen Ende des Leitungskörpers, wobei der proximale Kontakt mit dem elektrischen Verbinder (2124) elektrisch gekoppelt ist;
eine distale Elektrode an einem distalen Ende des Leitungskörpers;
einen elektrischen Leiter (2018, 2118, 2218, 2318), der den proximalen Kontakt mit der distalen Elektrode verbindet, wobei der proximale Kontakt elektrisch mit dem elektrischen Verbinder (2124) gekoppelt ist, um einen Leitungspfad vom elektrischen Verbinder (2124) zur distalen Elektrode zu bilden;
einen Temperatursensor (2548) innerhalb des Leitungskörpers und benachbart zu distalen Elektrode; und
einen Signalpfad, der sich proximal vom Temperatursensor (2548) durch den Leitungskörper erstreckt;
einen Schalter (2050) mit einem Steuereingang, einer ersten Verbindung, die elektrisch mit der Wärmesenke (2044, 2144, 2246, 2346, 2412) verbunden ist, und einer zweiten Verbindung, die elektrisch so mit dem Leitungspfad verbunden ist, dass sie parallel zu einem Abschnitt des Leitungspfads verläuft, der sich von der zweiten Verbindung zur distalen Elektrode erstreckt, sodass, wenn der Schalter (2050) geschlossen wird, der Leitungspfad über den Schalter (2054) zur Wärmesenke (2044, 2144, 2246, 2346, 2412) nebengeschlossen wird; und
eine Steuerung (2010, 2110, 2210, 2310) in Kommunikation mit dem Steuereingang des Schalters (2050) und in Kommunikation mit dem Signalpfad des Temperatursensors (2548), sodass die Steuerung ein Signal vom Temperatursensor (2548) empfängt, das die Temperatur darstellt, und den Schalter (2054) schließt, wenn die Temperatur, wie durch das empfangene Signal dargestellt, eine Schwelle überschreitet.

15. Implantierbares medizinisches System, umfassend:
eine implantierbare medizinische Vorrichtung, die umfasst:
einen Stimulationsmotor; und
einen elektrischen Verbinder (2736), der mit einem Ausgang des Stimulationsmotors elektrisch gekoppelt ist; und
eine implantierbare medizinische Leitung (2506, 2606, 2706, 2806), die umfasst:
einen Leitungskörper;
einen proximalen Kontakt an einem proximalen Ende des Leitungskörpers, wobei der proximale Kontakt mit dem elektrischen Verbinder (2736) elektrisch gekoppelt ist;
eine distale Elektrode an einem distalen Ende des Leitungskörpers;
einen elektrischen Leiter (2518, 2618, 2718, 2818), der den proximalen Kontakt mit der distalen Elektrode verbindet, wobei der proximale Kontakt elektrisch mit dem elektrische Verbinder (2736) gekoppelt ist, um einen Leitungspfad vom elektrischen Verbinder (2736) zur distalen Elektrode zu bilden;
einen Temperatursensor (2548) innerhalb des Leitungskörpers und benachbart zu distalen Elektrode; und
einen Signalpfad, der sich proximal vom Temperatursensor (2548) durch den Leitungskörper erstreckt;
einen Schalter (2554), der einen Steuereingang aufweist und im Leitungspfad in Reihe geschaltet ist, sodass, wenn der Schalter (2554) geöffnet wird, der Leitungspfad ein offener Kreis am Schalter (2554) ist; und
eine Steuerung (2510, 2610, 2710, 2810) in Kommunikation mit dem Steuereingang des Schalters (2554) und in Kommunikation mit dem Signalpfad des Temperatursensors (2548), sodass die Steuerung (2510, 2610, 2710, 2810) ein Signal vom Temperatursensor (2548) empfängt, das die Temperatur darstellt, und den Schalter (2554) öffnet, wenn die Temperatur, wie durch das empfangene Signal dargestellt, eine Schwelle überschreitet.

## Revendications

1. Procédé de réduction de chaleur au niveau d'une électrode (2049) sur un fil (2006, 2106, 2206, 2306, 2406) d'un système médical implantable (2000, 2100, 2200, 2300, 2400) pendant un balayage IRM, le fil (2006, 2106, 2206, 2306, 2406), comportant un ou plusieurs conducteurs de stimulation (2404) et un conducteur de dérivation (2052, 2240, 2320, 2408), le procédé comprenant :
la surveillance d'une température au niveau des une ou plusieurs électrodes (2049) pendant un balayage IRM ; et
lors de la détection que la température surveillée dépasse un seuil, le contournement alors, dans le conducteur de contournement (2052, 2240, 2320, 2408) du fil (2006, 2106, 2206, 2306, 2406), d'un courant électrique des un ou plusieurs conducteurs de stimulation (2404) vers un puit de chaleur (2044, 2144, 2246, 2346, 2412) situé à l'extérieur du système médical implantable (2200, 2300, 2400).

2. Procédé selon la revendication 1, le puit de chaleur (2044, 2144, 2246, 2346, 2412) comprenant un boîtier externe d'un dispositif médical implantable (2200) couplé au fil (2006, 2106, 2206, 2306, 2406) .

3. Procédé selon la revendication 1, le puit de chaleur (2044, 2144, 2246, 2346, 2412) comprenant une enceinte (2330) d'une unité de traitement de sonde couplée à un dispositif médical implantable du système médical implantable ou, le puit de chaleur (2044, 2144, 2246, 2346, 2412) étant positionné sur une surface extérieure d'un corps de fil du fil (2006, 2106, 2206, 2306, 2406).

4. Procédé selon la revendication 1, la surveillance de le température comprenant le fait d'inclure un capteur de température dans un corps de fil du fil (2006, 2106, 2206, 2306, 2406) et dans une position adjacente à l'électrode (2049).

5. Procédé selon la revendication 4, le fait d'inclure le capteur de température comprenant le fait d'inclure un câble de fibre optique dans le corps de fil et fixé au capteur de température de telle sorte que des signaux de capteur de température sont transférés sur le câble de fibre optique ou, le fait d'inclure le capteur de température comprenant le fait d'inclure une connexion électrique entre le capteur de température et un conducteur électrique dans le corps de fil.

6. Procédé selon la revendication 4, le fait d'inclure le capteur de température comprenant le fait d'inclure une connexion électrique entre le capteur de température et un conducteur électrique dans le corps de fil et, le conducteur électrique étant couplé électriquement à l'électrode.

7. Procédé selon la revendication 6, le conducteur électrique s'étendant vers un contact proximal sur le fil, spécifiquement le contact proximal se trouvant dans un trou d'une tête d'un dispositif médical implantable lorsque le fil est totalement inséré dans le trou.

8. Procédé de réduction d'une chauffe au niveau d'une électrode sur un fil (2506, 2606, 2706, 2806) d'un système médical implantable (2500, 2600, 2700, 2800) pendant un balayage IRM, comprenant :
la surveillance d'une température au niveau de l'électrode pendant un balayage IRM ; et
lors de la détection que la température surveillée dépasse un seuil, le blocage alors d'un courant électrique dans un conducteur de stimulation (2518, 2618, 2718, 2818) du fil en créant un circuit ouvert avec un conducteur de commande (2552) couplé à un commutateur (2554) connecté en série avec le conducteur de stimulation.

9. Procédé selon la revendication 8, le fil (2506, 2606, 2706, 2806) comportant une pluralité de conducteurs de stimulation, un commutateur distinct connecté en série avec chaque conducteur de stimulation de la pluralité étant présent, et le conducteur de commande étant couplé à chacun des commutateurs distincts.

10. Procédé selon la revendication 8, le commutateur (2554) étant compris dans une unité de traitement de sonde couplée au fil (2506, 2606, 2706, 2806) ou, le commutateur (2554) étant positionné dans un corps de fil du fil (2506, 2606, 2706, 2806).

11. Procédé selon la revendication 9, la surveillance de la température comprenant le fait d'inclure un capteur de température dans un corps de fil du fil et dans une position adjacente à l'électrode.

12. Procédé selon la revendication 11, le fait d'inclure le capteur de température comprenant le fait d'inclure un câble de fibre optique dans le corps de fil et fixé au capteur de température de telle sorte que des signaux de capteur de température sont transférés sur le câble de fibre optique ou, le fait d'inclure le capteur de température comprenant le fait d'inclure une connexion électrique entre le capteur de température et un conducteur électrique dans le corps de fil.

13. Procédé selon la revendication 11, le fait d'inclure le capteur de température comprenant le fait d'inclure une connexion électrique entre le capteur de température et un conducteur électrique dans le corps de fil et, le conducteur électrique étant couplé électriquement à l'électrode, spécifiquement le conducteur électrique s'étendant vers un contact proximal sur le fil (2506, 2606, 2706, 2806), plus spécifiquement, le contact proximal se trouvant dans un trou d'une tête d'un dispositif médical implantable lorsque le fil est totalement inséré dans le trou.

14. Système médical implantable, comprenant :
un puit de chaleur (2044, 2144, 2246, 2346, 2412) ;
un dispositif médical implantable qui comprend :
une machine de stimulation (2008, 2108, 2208, 2308) ; et
un connecteur électrique (2124) couplé électriquement à une sortie de la machine de stimulation (2008, 2108, 2208, 2308) ;
un fil (2006, 2106, 2206, 2306, 2406) médical implantable qui comprend :
un corps de fil ;
un contact proximal sur une extrémité proximale du corps de fil, le contact proximal étant couplé électriquement au connecteur électrique (2124) ;
une électrode distale sur une extrémité distale du corps de fil ;
un conducteur électrique (2018, 2118, 2218, 2318) qui interconnecte électriquement le contact proximal à l'électrode distale, avec le contact proximal couplé électriquement au connecteur électrique (2124) de manière à créer un chemin de conduction du connecteur électrique (2124) à l'électrode distale ;
un capteur de température (2548) dans le corps de fil et adjacent à l'électrode distale ; et
un chemin de signal s'étendant de manière proximale depuis le capteur de température (2548) à travers le corps de fil ;
un commutateur (2050) ayant une entrée de commande, une première connexion connectée électriquement au puit de chaleur (2044, 2144, 2246, 2346, 2412), et une deuxième connexion connectée électriquement au chemin de conduction de manière à être en parallèle avec une portion du chemin de conduction qui s'étend de la deuxième connexion à l'électrode distale de telle sorte que lorsque le commutateur (2050) est fermé le chemin de conduction est contourné vers le puit de chaleur (2044, 2144, 2246, 2346, 2412) via le commutateur (2054) ; et un contrôleur (2010, 2110, 2210, 2310) en communication avec l'entrée de commande du commutateur (2050) et en communication avec le chemin de signal du capteur de température (2548) de telle sorte que le contrôleur reçoit un signal représentatif de la température provenant du capteur de température (2548) et ferme le commutateur (2054) lorsque le température telle que représentée par le signal reçu dépasse un seuil.

15. Système médical implantable, comprenant :
un dispositif médical implantable qui comprend :
une machine de stimulation ; et
un connecteur électrique (2736) couplé électriquement à une sortie de la machine de stimulation ; et
un fil (2506, 2606, 2706, 2806) médical implantable qui comprend :
un corps de fil ;
un contact proximal sur une extrémité proximale du corps de fil, le contact proximal étant couplé électriquement au connecteur électrique (2736) ;
une électrode distale sur une extrémité distale du corps de fil ;
un conducteur électrique (2518, 2618, 2718, 2818) qui interconnecte électriquement le contact proximal à l'électrode distale, avec le contact proximal couplé électriquement au connecteur électrique (2736) de manière à créer un chemin de conduction du connecteur électrique (2736) à l'électrode distale ;
un capteur de température (2548) dans le corps de fil et adjacent à l'électrode distale ; et
un chemin de signal s'étendant de manière proximale depuis le capteur de température (2548) à travers le corps de fil ;
un commutateur (2554) ayant une entrée de commande et étant connecté en série dans le chemin de conduction de telle sorte que, lorsque le commutateur (2554) est ouvert, le chemin de conduction est un circuit ouvert au niveau du commutateur (2554) ; et
un contrôleur (2510, 2610, 2710, 2810) en communication avec l'entrée de commande du commutateur (2554) et en communication avec le chemin de signal du capteur de température de telle sorte que le contrôleur (2510, 2610, 2710, 2810) reçoit un signal représentatif de la température provenant du capteur de température (2548) et ouvre le commutateur (2554) lorsque le température telle que représentée par le signal reçu dépasse un seuil.
